(19) Europäisches Patentamt
European Patent Office
Office européen des brevets

(11) **EP 4 550 445 A1**

(12) # EUROPEAN PATENT APPLICATION
published in accordance with Art. 153(4) EPC

(43) Date of publication:
**07.05.2025 Bulletin 2025/19**

(21) Application number: **23850413.8**

(22) Date of filing: **02.08.2023**

(51) International Patent Classification (IPC):
***H01M 4/13** (2010.01)* ***H01M 4/66** (2006.01)*
***H01M 10/0525** (2010.01)* ***C08G 61/12** (2006.01)*
***C08L 65/00** (2006.01)* ***H01M 4/02** (2006.01)*

(52) Cooperative Patent Classification (CPC):
**C08G 61/12; C08L 65/00; H01M 4/02; H01M 4/13; H01M 4/66; H01M 10/0525**

(86) International application number:
**PCT/KR2023/011298**

(87) International publication number:
**WO 2024/029913 (08.02.2024 Gazette 2024/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **02.08.2022 KR 20220096262**

(71) Applicants:
- **LG Chem, Ltd.**
  **Yeongdeungpo-gu**
  **Seoul 07336 (KR)**
- **LG Energy Solution, Ltd.**
  **Seoul 07335 (KR)**

(72) Inventors:
- **KANG, Joon Koo**
  **Daejeon 34122 (KR)**
- **KWON, Soon Ho**
  **Daejeon 34122 (KR)**
- **KIM, Ki Hwan**
  **Daejeon 34122 (KR)**
- **KOH, Jong Kwan**
  **Daejeon 34122 (KR)**
- **KIM, Min Gyu**
  **Daejeon 34122 (KR)**
- **CHO, Woo Hyung**
  **Daejeon 34122 (KR)**
- **PARK, Sung Bin**
  **Daejeon 34122 (KR)**
- **PARK, Eun Kyoung**
  **Daejeon 34122 (KR)**
- **SONG, In Taek**
  **Daejeon 34122 (KR)**

(74) Representative: **Plasseraud IP**
**104 Rue de Richelieu**
**CS92104**
**75080 Paris Cedex 02 (FR)**

(54) **CURRENT COLLECTOR**

(57) The present application relates to a current collector, and a use thereof. The present application can provide a current collector capable of forming an electrode, which does not affect performance and operation of a secondary battery by exhibiting, in a normal state, excellent electrical characteristics including low resistance, and can ensure stability by blocking, in an abnormal state, energization of an electrode assembly through resistance increase, and a use thereof.

Fig. 1

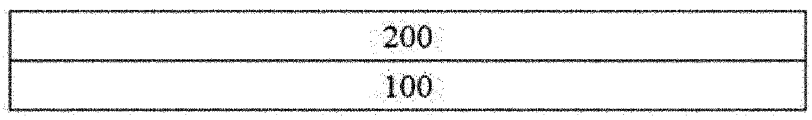


EP 4 550 445 A1

## Description

### Technical Field

This application claims the benefit of priority based on Korean Patent Application No. 10-2022-0096262 dated August 2, 2022, the disclosure of which is incorporated herein by reference in its entirety.
The present application relates to a current collector and a use thereof.

### Background Art

An energy storage technology has expanded application areas to mobile phones, tablet and notebook PCs, or electric vehicles, and the like.
As the data processing speed of mobile devices such as mobile phones or tablets increases and their used hours also increase, the development of secondary batteries with high energy density and operating potential, long cycle life, and low self-discharge rate is in progress.
In addition, as major developed countries suppress production of cars driven by internal combustion engines to alleviate global warming and air pollution, major automobile manufacturers also develop various electric vehicles, and thus the importance of secondary batteries having high energy density, high discharge voltage, and output stability increases as driving sources thereof.
However, according to the above trend, the occurrence frequency of ignition or explosion accidents due to overcharging, high-temperature exposure or outer shocks, and the like in devices or automobiles using secondary batteries as an energy source also increases.
As a main cause of such accidents, a short phenomenon, in which a positive electrode and a negative electrode inside an electrode assembly come into direct contact with each other mainly due to external stimuli, is known. When the secondary battery is overcharged or exposed to high temperatures or external stimuli, the short phenomenon can occur by the separator shrinkage due to the internal temperature increase of the secondary battery, or the internal structure destruction of the secondary battery due to outer shocks, and the like.
When the short phenomenon occurs, migration of lithium ions and electrons is concentrated through the region where the positive electrode and the negative electrode are in direct contact with each other, so that internal heat generation can be promoted. Accordingly, it is known that as gas or the like is generated inside the battery, the volume expands, and the risk of ignition increases.

### Disclosure

### Technical Problem

The present application relates to a current collector, and a use thereof. The present application is intended to provide a current collector capable of forming an electrode, which does not affect performance and operation of a secondary battery by exhibiting, in a normal state, excellent electrical characteristics including low resistance, and can ensure stability by blocking, in an abnormal state, energization of an electrode assembly through resistance increase, and a use thereof.

### Technical Solution

In this specification, the term room temperature means a natural temperature without heating or cooling, which may mean, for example, any one temperature in a range of 10°C to 30°C, or a temperature of about 23°C, about 25°C, or about 27°C or so.
Among the physical properties mentioned in this specification, when the measurement temperature affects the physical property, the relevant physical property is a physical property measured at room temperature, unless otherwise specified. The unit of temperature mentioned in this specification is Celsius (°C), unless otherwise specified.
In this specification, the term normal pressure means a natural pressure without pressurization or depressurization, which may usually mean a pressure of about 730 mmHg to 790 mmHg or so.
Among the physical properties mentioned in this specification, when the measurement pressure affects the physical property, the relevant physical property is a physical property measured at the above normal pressure, unless otherwise specified.
Among the physical properties mentioned in this specification, when the measurement humidity affects the physical property, the relevant physical property is a physical property measured at humidity under standard conditions, unless otherwise specified.

In this specification, the humidity under standard conditions means any relative humidity in a range of 40% to 60%, which means, for example, relative humidity of 40%, 45%, 50%, 55%, or 60% or so.

In this specification, the term normal state of an electrode or secondary battery means a normal operating state of the secondary battery, for example, a normal charging or discharging, or a storage state of the secondary battery.

In this specification, the term abnormal state of an electrode or secondary battery means a dangerous state in which abnormal heat generation or explosion, and the like occurs in a secondary battery, and the like.

The present application relates to an electrode current collector.

The electrode current collector of the present application may comprise a current collector main body, and a polymer layer formed on the current collector main body. The electrode current collector may be used to form an electrode. For example, an electrode formed using the electrode current collector may comprise the electrode current collector, and an active material layer formed on the polymer layer of the current collector. Figure 1 is a cross-sectional schematic diagram of an electrode current collector comprising a current collector main body (100) and a polymer layer (200), and Figure 2 is a cross-sectional schematic diagram showing an electrode in which an active material layer (300) is formed on the polymer layer (200) of the current collector.

As shown in the drawing, in the current collector or electrode, the current collector main body (100) and the polymer layer (200), and the polymer layer (200) and the active material layer (300) may also be in contact with each other. In some cases, other elements may also exist between the current collector main body (100) and the polymer layer (200), or between the polymer layer (200) and the active material layer (300). Also, in the drawing, the active material layer (300) is shown on only one side of the current collector main body (100), but the active material layer (300) may also be present on both sides of the current collector main body (100). In this case, the polymer layer (200) may also be present in two layers between each of the active material layers (300) present on both sides of the current collector body (100), and the current collector main body (100), and may also be present in one layer between any one of the active material layers (300) present on both sides, and the current collector main body (100).

The electrode formed by the electrode current collector of the present application may be a negative electrode (anode) or a positive electrode (cathode) applied to a secondary battery.

The polymer layer of the present application exhibits a so-called PTC (positive temperature coefficient) effect. Therefore, the polymer layer can variably control the migration of charges through the electrode depending on temperature.

By applying such a polymer layer, the electrode having the current collector of the present application can be applied to secondary batteries, and the like, thereby exhibiting, in a normal state, excellent electrical characteristics including low resistance, and securing, in an abnormal state, stability through resistance increase.

In order that the polymer layer is applied to an electrode to exhibit the above effect, the tendency of the PTC effect exhibited by the polymer layer must be controlled. The PTC effect is an effect in which the resistance increases in proportion to the temperature, and the temperature at the time point when the resistance increases by the PTC effect and the resistance of the polymer layer before the resistance increase affect the performance of the secondary battery. For example, if the PTC effect is excessively expressed at the temperature in the normal state, the performance of the secondary battery cannot be properly expressed before the stability is secured.

The polymer layer disclosed in this specification has a PTC effect, where such a PTC effect is adjusted so that it does not affect the performance of the secondary battery in a normal state, and the stability can be secured in an abnormal state.

For expression of such a PTC effect, crystallization characteristics of a conductive polymer in the polymer layer can be controlled. The electrical properties of the polymer layer are affected by the crystallinity of the conductive polymer, where if the crystallinity increases, the conductivity usually increases, and if the crystallinity of the conductive polymer is damaged due to exposure to high temperatures, the conductivity decreases, whereby the resistance increase can occur.

In the present application, as described below, by applying a conductive polymer having a relatively long hydrocarbon chain (long-chain hydrocarbon functional group), and controlling a drying or annealing temperature in a process of forming a polymer layer, an appropriate PTC effect (e.g., inducing the resistance increase of the polymer layer at the desired temperature (temperature of the battery in an abnormal state)) can be secured.

In one example, the polymer layer, or the current collector or electrode to which the polymer layer is applied may have a DC resistance of a certain level or less at 25°C. Accordingly, stable operation or storage of the secondary battery may be possible in a normal state. The upper limit of the DC resistance may be $10^4$ Ω·cm, 9500 Ω·cm, 9000 Ω·cm, 8500 Ω·cm, 8000 Ω·cm, 7500 Ω·cm, 7000 Ω·cm, 6500 Ω·cm, 6000 Ω·cm cm, 5500 Ω·cm, 5000 Ω·cm, 4500 Ω·cm, 4000 Ω·cm, 3500 Ω·cm, 3000 Ω·cm, 2500 Ω·cm, 2000 Ω·cm, 1500 Ω·cm, 1000 Ω·cm cm, 950 Ω·cm, 900 Ω·cm, 850 Ω·cm, 800 Ω·cm, 750 Ω·cm, 700 Ω·cm, 650 Ω·cm, 600 Ω·cm, 550 Ω·cm, 500 Ω·cm cm, 450 Ω·cm, 400 Ω·cm, or 350 Ω·cm or so, and the lower limit thereof may also be 10 Ω·cm, 50 Ω·cm, 100 Ω·cm, 150 Ω·cm, 200 Ω·cm, 250 Ω·cm, 300 Ω·cm, 350 Ω·cm, 400 Ω·cm, 450 Ω·cm, 500 Ω·cm, 550 Ω·cm,or 600 Ω·cm or so. The DC resistance may be within a range of less than or equal to, or less than any one of the above-described upper limits; or within a range of more than or equal to, or more than any one of the above-described lower limits while being less than or equal to, or less than any one of the above-described upper limits.

The DC resistance can be measured for a coin cell manufactured by applying the polymer layer as described in Examples of this specification.

In one example, the polymer layer, or the current collector or electrode to which the polymer layer is applied may have an AC impedance resistance of a certain level or less at 25°C. Accordingly, stable operation or storage of the secondary battery may be possible in a normal state. The upper limit of the AC impedance resistance may be $10^3$ Ω, 950 Ω, 900 Ω, 850 Ω, 800 Ω, 750 Ω, 700 Ω, 650 Ω, 600 Ω, 550 Ω, 500 Ω, 450 Ω, 400 Ω, 350 Ω, 300 Ω, 250 Ω, 200 Ω, 150 Ω, 100 Ω, 95 Ω, 90 Ω, 85 Ω, 80 Ω, 75 Ω, 70 Ω, 65 Ω, 60 Ω, 55 Ω, or 50 Ω or so, and the lower limit thereof may also be 10 Ω, 15 Ω, 20 Ω, 25 Ω, 30 Ω, 35 Ω, 40 Ω, 450 Ω, 50 Ω, 55 Ω, 60 Ω, 65 Ω, 70 Ω, 75 Ω, 80 Ω, or 85 Ω or so. The AC impedance resistance may be within a range of less than or equal to, or less than any one of the above-described upper limits; or within a range of more than or equal to, or more than any one of the above-described lower limits while being less than or equal to, or less than any one of the above-described upper limits. The AC impedance resistance can be measured for a coin cell manufactured by applying the polymer layer as described in Examples of this specification.

By exhibiting the DC resistance and/or AC impedance resistance, the secondary battery to which the electrode current collector is applied can be stably operated and stored in a normal state.

The current collector of the present application can secure stability by exhibiting the increased resistance in an abnormal state, and blocking energization of the electrode assembly therethrough.

The polymer layer, or the current collector or electrode to which the polymer layer is applied may exhibit a characteristic that ΔR1 of Equation 1 below is in a certain level or more.

[Equation 1]

$$\Delta R1 = \mathrm{Max}\{(R_{n+5}/R_n)/5\}$$

In Equation 1, $R_n$ is the DC resistance at any temperature n°C within a range of 25°C to 135°C, and $R_{n+5}$ is the DC resistance at a temperature ((n+5)°C) 5°C higher than the temperature n°C, and Max{($R_{n+5}$/$R_n$)/5} is the maximum value among the ($R_{n+5}$/$R_n$)/5 values confirmed within the temperature range of 25°C to 135°C.

A method of measuring ΔR1 in Equation 1 is explained in Examples. In the method for identifying ΔR1, an initial temperature is 25°C and a final temperature is 135°C. The $R_{n+5}$ and $R_n$ are identified by measuring the DC resistance at each temperature while increasing the temperature by 5°C from the initial temperature of 25°C. For example, when n is 90, $R_{95}$/$R_{90}$ is the ratio of the DC resistance at 95°C to the DC resistance at 90°C. The matter that ΔR1 exhibits a certain level or more at any temperature within the temperature range of 25°C to 135°C means that the resistance increases relatively rapidly at any temperature within the temperature range.

The lower limit of ΔR1 may be 100Ω·cm/°C, 150Ω·cm/°C, 200Ω·cm/°C, 250Ω·cm/°C, 300Ω·cm/°C, 350Ω·cm/°C, or 400Ω·cm/°C, and the upper limit thereof may also be 1,000 Ω·cm/°C, 950 Ω·cm/°C, 900 Ω·cm/°C, 850 Ω·cm/°C, 800 Ω·cm/°C, 750 Ω·cm/°C, 700 Ω·cm/°C, 650 Ω·cm/°C, 600 Ω·cm/°C, 550 Ω·cm/°C, 500 Ω·cm/°C, 450 Ω·cm/°C, 400 Ω·cm/°C, 350 Ω·cm/°C, 300 Ω·cm/°C, or 250 Ω·cm/°C or so. The ΔR1 may be within a range of more than or equal to, or more than any one of the above-described lower limits; or within a range of less than or equal to, or less than any one of the above-described upper limits while being more than or equal to, or more than any one of the above-described lower limits.

The temperature at which ΔR1 in the above range is identified, that is, the temperature at $R_n$ in Equation 1, may be the range. The above temperature range is very important in terms of ensuring stable operation and stability of the secondary battery. That is, if the temperature is in the normal temperature range of the secondary battery, the temperature increase at the relevant temperature adversely affects the performance of the secondary battery. The lower limit of the temperature at $R_n$ in Equation 1 above may be 80°C, 80°C, 81°C, 82°C, 83°C, 84°C, 85°C, 86°C, 87°C, 88°C, 89°C, 90°C, 91°C, 92°C, 93°C, 94°C, or 95°C or so, and the upper limit thereof may be 200°C, 190°C, 180°C, 170°C, 160°C, 150°C, 140°C, 130°C, 120°C, 110°C, 100°C, or 90°C or so. The temperature may be within a range of more than, or more than or equal to the above-described lower limits; or within a range of less than or equal to, or less than any one of the above-described upper limits while being more than or equal to, or more than any one of the above-described lower limits.

By controlling so that the resistance increase occurs in the above temperature range, it is possible to enable stable operation and storage in a normal state and stable blocking of energization in an abnormal state. For example, even though the storage of secondary batteries is performed at a relatively high temperature, stable storage is possible through adjustment of the temperature range.

The polymer layer, or the current collector or electrode to which the polymer layer is applied may exhibit a characteristic that ΔR2 of Equation 2 below is in a certain level or more.

[Equation 2]

$$\Delta R2 = Max\{(R_{z+5}/R_z)/5\}$$

In Equation 2, $R_z$ is the AC impedance resistance at any temperature n°C within a range of 25°C to 135°C, and $R_{z+5}$ is the AC impedance resistance at a temperature ((n+5)°C) 5°C higher than the temperature n°C, and Max $\{(R_{z+5}/R_z)/5\}$ is the maximum value among the $(R_{z+5}/R_z)/5$ values confirmed within the temperature range of 25°C to 135°C.

A method of measuring ΔR2 in Equation 2 above is explained in Examples. In the method for identifying ΔR2, an initial temperature is 25°C and a final temperature is 135°C. The $R_{z+5}$ and $R_z$ are identified by measuring the AC impedance resistance at each temperature while increasing the temperature by 5°C from the initial temperature of 25°C. For example, when n is 90, $R_{95}/R_{90}$ is the ratio of the AC impedance resistance at 95°C to the AC impedance resistance at 90°C. The matter that ΔR2 exhibits 10 Ω/°C or more at any temperature within the temperature range of 25°C to 135°C means that the resistance of the polymer layer or electrode increases relatively rapidly at any temperature within the temperature range.

The lower limit of ΔR2 may be 10Ω/°C, 12Ω/°C, 14Ω/°C, 16/°C, 18Ω/°C, 20Ω/°C, 22Ω/°C, 24 Ω/°C, 26 Ω/°C, 28 Ω/°C, 30 Ω/°C, or 33 Ω/°C, and the upper limit thereof may be 100 Ω/°C, 95 Ω/°C, 90 Ω/°C, 85 Ω/°C, 80 Ω/°C, 75 Ω/°C, 70 Ω/°C, 65 Ω/°C, 60 Ω/°C, 55 Ω/°C, 50 Ω/°C, 45 Ω/°C, 40 Ω/°C, 35 Ω/°C, 30 Ω/°C, or 25 Ω/°C or so. The range of ΔR2 may be within a range of more than, or more than or equal to the above-described lower limits; or within a range of less than or equal to, or less than any one of the above-described upper limits while being more than or equal to, or more than any one of the above-described lower limits.

It is possible to ensure stability by exhibiting the resistance increase in an abnormal state through securing the above characteristics, and blocking energization therethrough.

As in the case of the equation, the temperature at which ΔR2 is identified, that is, the temperature range at $R_z$, is very important. The lower limit of the temperature may be 80°C, 81°C, 82°C, 83°C, 84°C, 85°C, 86°C, 87°C, 88°C, 89°C, 90°C, 91°C, 92°C, 93°C, 94°C, or 95°C or so, and the upper limit thereof may be 200°C, 190°C, 180°C, 170°C, 160°C, 150°C, 140°C, 130°C, 120°C, 110°C, 100°C, or 90°C or so. The temperature may be within a range of more than, or more than or equal to the above-described lower limits; or within a range of less than or equal to, or less than any one of the above-described upper limits while being more than or equal to, or more than any one of the above-described lower limits.

By controlling so that the resistance increase occurs in the above temperature range, it is possible to enable stable operation and storage in a normal state and stable blocking of energization in an abnormal state. For example, even though the storage of secondary batteries is performed at a relatively high temperature, stable storage is possible through adjustment of the temperature range.

The polymer layer, or the current collector or electrode to which the polymer layer is applied, or the secondary battery to which the foregoing is applied may exhibit a characteristic that an absolute value of ΔR3 in Equation 3 below is within a certain range.

[Equation 3]

$$\Delta R3 = 100 \times (C_1\text{-}C_2)/C_1$$

In Equation 3, $C_1$ is a discharged capacity at room temperature (about 25°C), and $C_2$ is a discharged capacity after storage at 70°C for 60 hours. $C_1$ and $C_2$ in Equation 3 are the discharged capacities measured for the coin cell to which the polymer layer is applied, and the specific method for measuring the same is summarized in Examples.

The upper limit of the absolute value of ΔR3 in Equation 3 may be 10%, 9.5%, 9%, 8.5%, 8%, 7.5%, 7%, 6.5%, 6%, 5.5%, 5%, 4.5%, 4%, 3.5%, 3%, 2.5%, 2%, 1.5%, 1%, or 0.5% or so, and the lower limit thereof may be 0%, 0.5%, or 1.5% or so. The absolute value of ΔR3 may be within a range of less than or equal to, or less than any one of the above-described upper limits; or within a range of less than or equal to, or less than any one of the above-described upper limits while being more than or equal to, or more than any one of the above-described lower limits. ΔR3 in the above range means that stable operation and storage are possible even when the secondary battery is operated and stored at a relatively high temperature within the normal state range.

The polymer layer, or the current collector or electrode to which the polymer layer is applied, or the secondary battery to which the foregoing is applied may exhibit a characteristic in which an absolute value of ΔR4 in Equation 4 below is 50% or more.

[Equation 4]

$$\Delta R4 = 100 \times (C_1 - C_3)/C_1$$

In Equation 4, $C_1$ is a discharged capacity at room temperature (about 25°C), and $C_3$ is a discharged capacity after storage at 130°C for 10 minutes.

$C_1$ and $C_3$ in Equation 4 are the discharged capacities measured for the coin cell to which the polymer layer is applied, and the specific method of measuring the same is summarized in Examples.

The lower limit of the absolute value of ΔR4 in Equation 4 may be 50%, 52%, 54%, 56%, 58%, 60%, 62%, 64%, 66%, 68%, 70%, 72%, 74%, or 76%, and the upper limit thereof may also be 200%, 180%, 160%, 140%, 120%, 100%, 95%, 90%, 85%, 80%, 75%, 70%, 65%, or 60% or so. The absolute value of ΔR4 may be within a range of more than or equal to, or more than any one of the above-described lower limits; or within a range of less than or equal to, or less than any one of the above-described upper limits while being more than or equal to, or more than any one of the above-described lower limits. ΔR4 in the above range means that stability is effectively secured by the resistance increase of the secondary battery in an abnormal state.

The above characteristics can be achieved through the introduction of a polymer layer to be described below.

The current collector main body is not particularly limited, and one commonly used as a current collector main body for the positive electrode or negative electrode can be used.

If the current collector main body has conductivity without causing chemical changes in applied devices such as secondary batteries, the type, size, and shape thereof, and the like are not particularly limited. An example of a material that can be used as the current collector main body may include copper, aluminum, stainless steel, nickel, titanium, or calcined carbon, and the like, or may be exemplified by a material, and the like in which the surface of copper, aluminum, or stainless steel is surface-treated with carbon, nickel, titanium, or silver, and the like. The current collector main body may be in the form of a film, sheet, foil, net, porous body, foam, or non-woven fabric, and the like, which comprises the above material. In some cases, a known surface treatment may also be performed on the surface of the current collector main body to improve adhesive force to other layers such as a polymer layer or an active material layer.

Such a current collector main body may typically have a thickness within a range of 3 μm to 500 μm, but is not limited thereto.

A polymer layer is present on one or both sides of the current collector main body.

In this specification, the term polymer layer refers to a layer comprising a polymer. For example, the lower limit of the content of the polymer contained in the polymer layer may be 30 weight%, 35 weight%, 40 weight%, 45 weight%, 50 weight%, 55 weight%, 60 weight%, 65 weight%, 70 weight%, 75 weight%, 80 weight%, 85 weight%, 90 weight%, or 95 weight% or so, and the upper limit thereof may be 100 weight%, 95 weight%, 90 weight%, 85 weight%, 80 weight%, 75 weight%, 70 weight%, 65 weight%, 60 weight%, 55 weight%, or 50 weight% or so. The content is the content of the polymer based on the total weight of the polymer layer. The content may be within a range of more than or equal to, or more than any one of the above-described lower limits; or within a range of more than or equal to, or more than any one of the above-described lower limits while being less than or equal to, or less than any one of the above-described upper limits.

The polymer layer may not be the so-called active material layer of the electrode. Therefore, the content of the electrode active material in the polymer layer may be controlled. For example, the upper limit of the content of the electrode active material in the polymer layer may be 10 weight%, 9 weight%, 8 weight%, 7 weight%, 6 weight%, 5 weight%, 4 weight%, 3 weight%, 2 weight%, 1 weight%, 0.5 weight%, 0.1 weight%, 0.05 weight%, 0.01 weight%, 0.005 weight%, or 0.001 weight% or so, and the lower limit thereof may be 0 weight%. The content is the content of the polymer based on the total weight of the polymer layer. The content may be within a range of less than or equal to, or less than any one of the above-described upper limits; or within a range of more than or equal to, or more than any one of the above-described lower limits while being less than or equal to, or less than any one of the above-described upper limits. Specific types of the electrode active material will be described below.

The polymer included in the polymer layer may be a conductive polymer. As is well known, a conductive polymer is a polymer that exhibits conductivity through conjugated systems of polymer chains and/or doping, and the like.

The conductive polymer may be a conductive copolymer. The conductive copolymer is a type of conductive polymer and is distinguished from the conductive polymer in the form of a homopolymer in that it is a conductive polymer containing two or more monomer units.

Using a specific conductive copolymer to be described below, it is possible to effectively secure the above-mentioned properties through effective control of the crystallinity and oxidation potential of the relevant copolymer.

The oxidation potential of the conductive copolymer or the polymer layer may be adjusted depending on the purpose. A method of measuring the oxidation potential is summarized in Examples of this specification. The oxidation potential varies depending on the electrode and electrolyte used for measurement, where in this specification, the oxidation potential is the oxidation potential measured based on lithium and lithium ions ($Li/Li^+$). In the present application,

it is possible to secure the desired characteristics by controlling the oxidation potential measured by the measurement method described in Examples below.

The lower limit of the oxidation potential may be 2V, 2.1V, 2.2V, 2.3V, 2.4V, 2.5V, 2.6V, 2.7V, 2.8V, 2.9V, 3V, 3.1V, 3.2V, 3.3V, 3.4V, 3.5V, 3.6V, or 3.7V or so, and the upper limit thereof may also be 6V, 5.5V, 5V, 4.9V, 4.8V, 4.7V, 4.6V, 4.5V, 4.4V, 4.3V, 4.2V, 4.1V, 4.0V, 3.9V, 3.8V, 3.7V, 3.6V, or 3.5V or so. The oxidation potential may be within a range of less than or equal to, or less than any one of the above-described upper limits; within a range of more than or equal to, or more than any one of the above-described lower limits; or within a range of more than or equal to, or more than any one of the above-described lower limits while being less than or equal to, or less than any one of the above-described upper limits. By adjusting the oxidation potential to such a range, it is possible to effectively secure the desired characteristics.

The conductive copolymer may have a weight average molecular weight within a predetermined range. The lower limit of the weight average molecular weight of the conductive copolymer may be 30,000 g/mol, 35,000 g/mol, 40,000 g/mol, 45,000 g/mol, 50,000 g/mol, 55,000 g/mol, or 60,000 g/mol or so, and the upper limit thereof may be 200,000 g/mol, 150,000 g/mol, 100,000 g/mol, 95,000 g/mol, 90,000 g/mol, 85,000 g/mol, 80,000 g/mol, 75,000 g/mol, 70,000 g/mol, 65,000 g/mol, 60,000 g/mol, 55,000 g/mol, or 50,000 g/mol or so. The weight average molecular weight may be within a range of less than or equal to, or less than any one of the above-described upper limits; within a range of more than or equal to, or more than any one of the above-described lower limits; or within a range of more than or equal to, or more than any one of the above-described lower limits while being less than or equal to, or less than any one of the above-described upper limits. By using the conductive copolymer having such a weight average molecular weight, it is possible to effectively form the polymer layer and electrode with desired characteristics.

The molecular weight distribution of the conductive copolymer, that is, the ratio of the weight average molecular weight (Mw) to the number average molecular weight (Mn), may be within a predetermined range. The lower limit of the molecular weight distribution may be 2, 2.5, 3, 3.5, or 4 or so, and the upper limit thereof may be 10, 9, 8, 7.5, 7, 6.5, 6, 5.5, 5, 4.5, 4, or 3.5 or so. The molecular weight distribution may be within a range of less than or equal to, or less than any one of the above-described upper limits; within a range of more than or equal to, or more than any one of the above-described lower limits; or within a range of more than or equal to, or more than any one of the above-described lower limits while being less than or equal to, or less than any one of the above-described upper limits. By using the conductive copolymer having such a molecular weight distribution, it is possible to effectively form the polymer layer and electrode with desired characteristics.

The conductive copolymer may be a thiophene copolymer.

In this specification, the term thiophene copolymer means a copolymer containing the thiophene monomer unit in a certain level or more. The lower limit of the molar ratio of the thiophene monomer unit relative to the mole number of all monomer units included in the thiophene copolymer may be 50 mol%, 55 mol%, 60 mol%, 65 mol%, 70 mol%, 75 mol%, 80 mol%, 85 mol%, or 90 mol% or so, and the upper limit thereof may be 100 mol%, 95 mol%, or 90 mol% or so. The range may be within a range of more than or equal to, or more than any one of the above-described lower limits; or within a range of more than or equal to, or more than any one of the above-described lower limits while being less than or equal to, or less than any one of the above-described upper limits.

In this specification, the monomer unit means a form in which a monomer is polymerized and included in a polymer, and the thiophene monomer is a thiophene-based monomer, which means a monomer including a thiophene skeleton.

The conductive copolymer of the present application may contain a long-chain hydrocarbon functional group, or a monomer unit (hereinafter, may be referred to as a second unit) having the long-chain hydrocarbon functional group. The monomer having the long-chain hydrocarbon functional group may be a thiophene monomer.

In this specification, the term long-chain hydrocarbon functional group means a monovalent hydrocarbon group having carbon atoms in a certain level or more, or a monovalent functional group containing the hydrocarbon structure having carbon atoms in a certain level or more.

For example, the lower limit of the number of carbon atoms present in the long-chain hydrocarbon functional group (i.e., the number of carbon atoms in the monovalent hydrocarbon group or hydrocarbon structure) may be 3, 4, 5, 6, 7, 8, 9, 10, 11, or 12 or so, and the upper limit thereof may be 30, 29, 28, 27, 26, 25, 24, 23, 22, 21, 20, 19, 18, 17, 16, 15, 14, 13, 12, 11, 10, 9, 8, 7, 6, 5, or 4 or so. The number of carbon atoms may be within a range of more than or equal to, or more than any one of the above-described lower limits; or within a range of more than or equal to, or more than any one of the above-described lower limits while being less than or equal to, or less than any one of the above-described upper limits.

The number of carbon atoms may be the number of carbon atoms of a linear hydrocarbon chain present in the long-chain hydrocarbon functional group. That is, the monovalent hydrocarbon group or hydrocarbon structure present in the long-chain hydrocarbon functional group may have a linear structure or a branched structure, where even in the case of the branched structure, the number of carbon atoms constituting the longest linear chain in the relevant branched structure may be within the above range. For example, if the branched structure is a 2-ethylhexyl group, the number of carbon atoms constituting the longest linear chain is 6.

An example of the long-chain hydrocarbon functional group may be exemplified by one or more selected from the

group consisting of an alkyl group, an alkenyl group, an alkynyl group, an alkoxy group, an alkylcarbonyl group, and an alkylcarbonyloxy. In a suitable example, the long-chain hydrocarbon functional group may be an alkyl group and/or an alkoxy group.

The number of carbon atoms in the alkyl group, alkenyl group and alkynyl group, and the alkyl group present in the alkoxy group, alkylcarbonyl group and alkylcarbonyloxy may be in the range of the number of carbon atoms present in the long-chain hydrocarbon functional group (i.e., the number of carbon atoms in the monovalent hydrocarbon group or hydrocarbon structure).

For example, the alkyl group, alkenyl group and alkynyl group, and the alkyl group present in the alkoxy group, alkylcarbonyl group and alkylcarbonyloxy may have a linear or branched structure, where in the case of the branched chain, the number of carbon atoms constituting the longest linear chain in the relevant branched structure may be within the above range.

The alkyl group, alkenyl group, alkynyl group, alkoxy group, alkylcarbonyl group, or alkylcarbonyloxy, which is a long-chain hydrocarbon functional group, may also be optionally substituted with one or more substituents.

Such a long-chain hydrocarbon functional group is a functional group capable of imparting appropriate mobility to the monomer during the polymerization process of the conductive copolymer or the conductive copolymer itself. The monomer containing such a long-chain hydrocarbon functional group imparts appropriate mobility to a monomer mixture, and diffuses within the monomer mixture to enable polymerization to occur with excellent efficiency. Also, the conductive copolymer having the long-chain hydrocarbon functional group can enable a polymer layer to be formed stably and uniformly between the current collector main body and the active material layer through appropriate mobility.

The long-chain hydrocarbon functional group is appropriately oriented during the drying or annealing process applied in the process of forming the polymer layer, whereby it can also impart the suitable PTC effect to the copolymer.

Upon application of a certain amount of heat energy, the long-chain hydrocarbon functional group vibrates due to the heat, and dedoping of anions bound to the copolymer are promoted by this vibration (thermal vibration), whereby the resistance increase is induced. The temperature at which the thermal vibration occurs can be controlled by the length and/or amount of the long-chain hydrocarbon functional group. For example, under the same temperature, the thermal vibration of a relatively long chain is greater than that of a relatively short chain, whereby the long chain can induce a resistance increase effect at a relatively low temperature. Therefore, the desired PTC effect can be set through control of the length and/or ratio of the long-chain hydrocarbon functional group.

For example, to appropriately implement the above effect, the molar ratio of the monomer unit (first unit) having the long-chain hydrocarbon functional group relative to the mole number of all monomer units of the conductive copolymer may be adjusted. For example, the lower limit of the molar ratio may be 50 mol%, 55 mol%, 60 mol%, 65 mol%, 70 mol%, 75 mol%, 80 mol%, 85 mol%, or 90 mol% or so, and the upper limit thereof may be 99 mol%, 95 mol%, 90 mol%, 85 mol%, or 80 mol% or so. The ratio may be within a range of more than or equal to, or more than any one of the above-described lower limits; or within a range of more than or equal to, or more than any one of the above-described lower limits while being less than or equal to, or less than any one of the above-described upper limits.

The conductive copolymer may contain a polar functional group, or a monomer unit (hereinafter, may be referred to as a second unit) having the polar functional group, along with the long-chain hydrocarbon functional group, or the first unit. The monomer having the polar functional group may be a thiophene monomer.

In this specification, the term polar functional group is a functional group containing one or two or more polar atoms, for example, oxygen and/or nitrogen. An example of such a functional group includes a carboxyl group, a hydroxy group, an amino group, a cyano group, a nitro group, an ether group, or a functional group of Formula 3 below, but is not limited thereto. In one example, as the polar functional group, a functional group of Formula 3 below may be applied.

[Formula 3]

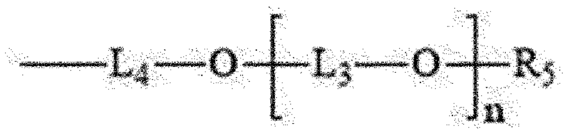

In Formula 3, $L_4$ is a single bond, an alkylene group or an alkylidene group, $L_3$ is an alkylene group or an alkylidene group, $R_5$ is hydrogen or an alkyl group, and n is an arbitrary number.

In Formula 3, the matter that $L_4$ is a single bond means a form in which $L_4$ does not exist, and the oxygen atom between $L_4$ and $L_3$ is directly connected to a monomer.

In one example, the alkyl group of $R_5$ in Formula 3 may be an alkyl group with 1 to 20 carbon atoms, 1 to 16 carbon atoms, 1 to 12 carbon atoms, 1 to 8 carbon atoms, or 1 to 4 carbon atoms, or may be a methyl group or an ethyl group. The alkyl group may be linear, branched, or cyclic, and may be suitably linear or branched. The alkyl group may be optionally

substituted with one or more substituents.

In this specification, the term alkylene group means a divalent functional group formed by removing hydrogen atoms from two different carbon atoms, respectively, in an alkane, and the term alkylidene group means a divalent functional group formed by removing two hydrogen atoms from one carbon atom in an alkane.

In one example, the alkylene groups of $L_3$ and $L_4$ in Formula 3 may each be an alkylene group with 2 to 20 carbon atoms, 2 to 16 carbon atoms, 2 to 12 carbon atoms, 2 to 8 carbon atoms, or 2 to 4 carbon atoms, or may be an ethylene group or a propylene group. The alkylene group may be linear, branched, or cyclic, and may be suitably linear or branched. The alkylene group may be optionally substituted with one or more substituents.

In one example, the alkylidene groups of $L_3$ and $L_4$ in Formula 3 may each be an alkylidene group with 1 to 20 carbon atoms, 1 to 16 carbon atoms, 1 to 12 carbon atoms, 1 to 8 carbon atoms, or 1 to 4 carbon atoms, or may be a methylidene group, an ethylidene group or a propylidene group. The alkylidene group may be linear, branched, or cyclic, and may be suitably linear or branched. The alkylidene group may be optionally substituted with one or more substituents.

In Formula 3, the lower limit of n may be 1, 2, 3, or 4, and the upper limit thereof may be 10, 9, 8, 7, 6, 5, 4, or 3 or so. The n may be within a range of more than or equal to, or more than any one of the above-described lower limits while being less than or equal to, or less than any one of the above-described upper limits.

Through the application of the polar functional group, the polymer layer may be bonded to other layers to have an appropriate bonding force, and the desired protective function may be efficiently achieved by uniformly forming the conductive copolymer layer.

In the conductive copolymer, the mole numbers of the polar functional group and the long-chain hydrocarbon functional group may be controlled to ensure an appropriate effect.

For example, the lower limit of the ratio (M1/M2) of the mole number (M1) of the long-chain hydrocarbon functional group to the mole number (M2) of the polar functional group in the conductive copolymer may be 1, 1.5, 2, 2.5, 3, 3.5, 4, 4.5, 5, 5.5, 6, 6.5, 7, 7.5, 8, or 8.5 or so, and the upper limit thereof may be 20, 19, 18, 17, 16, 15, 14, 13, 12, 11, 10, 9, 8, 7, 6, 5, or 4 or so. The ratio M1/M2 may be within a range of less than or equal to, or less than any one of the above-described upper limits; within a range of more than or equal to, or more than any one of the above-described lower limits; or within a range of more than or equal to, or more than any one of the above-described lower limits while being less than or equal to, or less than any one of the above-described upper limits.

The lower limit of the ratio (M1/M2) of the first unit (M1) to the mole number (M2) of the second unit in the conductive copolymer may be 1, 1.5, 2, 2.5, 3, 3.5, 4, 4.5, 5, 5.5, 6, 6.5, 7, 7.5, 8, or 8.5 or so, and the upper limit thereof may be 20, 19, 18, 17, 16, 15, 14, 13, 12, 11, 10, 9, 8, 7, 6, 5, or 4 or so. The ratio M1/M2 may be within a range of less than or equal to, or less than any one of the above-described upper limits; within a range of more than or equal to, or more than any one of the above-described lower limits; or within a range of more than or equal to, or more than any one of the above-described lower limits while being less than or equal to, or less than any one of the above-described upper limits.

The lower limit of the ratio of the total mole number of the first and second units to the total mole number of all monomer units of the conductive copolymer may be 50 mol%, 55 mol%, 60 mol%, 65 mol%, 70 mol%, 75 mol%, 80 mol%, 85 mol%, 90 mol%, or 95 mol% or so, and the upper limit thereof may be 100 mol%, 95 mol%, or 90 mol% or so. The ratio may be within a range of more than or equal to, or more than any one of the above-described lower limits; or within a range of more than or equal to, or more than any one of the above-described lower limits while being less than or equal to, or less than any one of the above-described upper limits.

The conductive copolymer may include a monomer unit of Formula 1 below as the thiophene monomer unit.

[Formula 1]

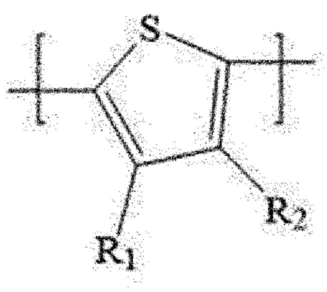

In Formula 1, $R_1$ and $R_2$ are each independently hydrogen, the polar functional group, or the long-chain hydrocarbon functional group, but at least one of $R_1$ and $R_2$ may be the polar functional group or the long-chain hydrocarbon functional group.

In another example, $R_1$ and $R_2$ in Formula 1 may be connected to each other to form a divalent functional group of

Formula 2 below.

[Formula 2]

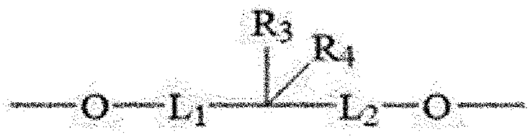

In Formula 2, each oxygen atom may be bonded to the carbon atom to which $R_1$ is connected and the carbon atom to which $R_2$ is connected in Formula 1.

In Formula 2, $L_1$ and $L_2$ are each independently a single bond, an alkylene group, or an alkylidene group, and $R_3$ and $R_4$ are each independently hydrogen, a polar functional group, or a long-chain hydrocarbon functional group, but at least one of $R_3$ and $R_4$ is a polar functional group or a long-chain hydrocarbon functional group.

The specific types of the polar functional group and the long-chain hydrocarbon functional group in Formulas 1 and 2 are as described above.

Also, the meaning that $L_1$ or $L_2$ in Formula 2 is a single bond is the same as that of $L_4$ in Formula 3.

The specific types of the alkylene group and the alkylidene group of $L_1$ and $L_2$ in Formula 2 are the same as the alkylene group and the alkylidene group in Formula 3.

The lower limit of the molar ratio of the monomer unit of Formula 1 relative to the mole number of all monomer units included in the conductive copolymer may be 50 mol%, 55 mol%, 60 mol%, 65 mol%, 70 mol%, 75 mol%, 80 mol%, 85 mol%, or 90 mol% or so, and the upper limit thereof may be 100 mol%, 95 mol%, or 90 mol% or so. The range may be within a range of more than or equal to, or more than any one of the above-described lower limits; or within a range of more than or equal to, or more than any one of the above-described lower limits while being less than or equal to, or less than any one of the above-described upper limits.

The conductive copolymer may simultaneously include a monomer unit of Formula 4 below and a monomer unit of Formula 5 below as monomer units.

The monomer unit of Formula 4 below is an example of the above-described first unit, and the monomer unit of Formula 5 below is an example of the above-described second unit.

[Formula 4]

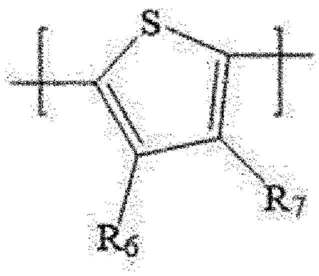

In Formula 4, $R_6$ and $R_7$ are each independently hydrogen or the long-chain hydrocarbon functional group, and at least one of $R_6$ and $R_7$ is the long-chain hydrocarbon functional group. Specific details about the long-chain hydrocarbon functional group are as described above.

In another example, $R_6$ and $R_7$ of Formula 4 may be connected to each other to form a divalent functional group of Formula 6 below.

[Formula 6]

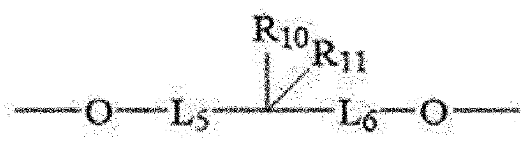

In Formula 6, $L_5$ and $L_6$ are each independently a single bond, an alkylene group, or an alkylidene group, and $R_{10}$

and $R_{11}$ are each independently hydrogen or the long-chain hydrocarbon functional group, but at least one of $R_{10}$ and $R_{11}$ is the long-chain hydrocarbon functional group. The specific types of the long-chain hydrocarbon functional groups in Formulas 4 and 6 are as described above.

Also, in Formula 6, the meaning of the single bond, and the specific types of the alkylene group and the alkylidene group are the same as in Formula 3.

[Formula 5]

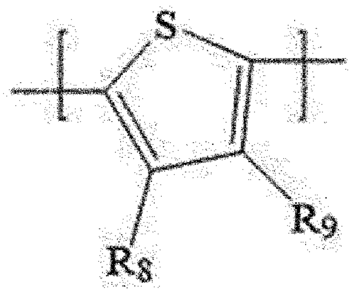

In Formula 5, $R_8$ and $R_9$ are each independently hydrogen or the polar functional group, but at least one of $R_8$ and $R_9$ may be the polar functional group.

In another example, $R_8$ and $R_9$ of Formula 5 may be connected to each other to form a divalent functional group of Formula 7 below.

[Formula 7]

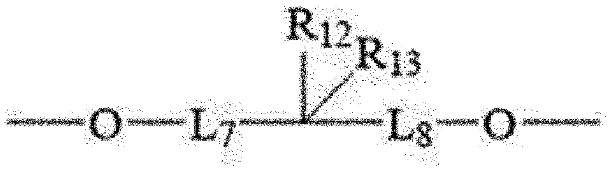

In Formula 7, $L_7$ and $L_8$ are each independently a single bond, an alkylene group, or an alkylidene group, and $R_{12}$ and $R_{13}$ are each independently hydrogen or the polar functional group, but at least one of $R_{12}$ and $R_{13}$ may be the polar functional group.

The specific types of the polar functional groups in Formulas 5 and 7 are as described above.

In addition, the meaning of the single bond in Formula 7, and the specific types of the alkylene group and the alkylidene group are the same as those in Formula 3.

For example, the lower limit of the ratio of the mole number of the monomer unit of Formula 4 above to the mole number of the total monomer units of the conductive copolymer may be 50 mol%, 55 mol%, 60 mol%, 65 mol%, 70 mol%, 75 mol%, 80 mol%, 85 mol%, or 90 mol% or so, and the upper limit thereof may be 99 mol%, 95 mol%, 90 mol%, 85 mol%, or 80 mol% or so. The ratio may be within a range of more than or equal to, or more than any one of the above-described lower limits; or within a range of more than or equal to, or more than any one of the above-described lower limits while being less than or equal to, or less than any one of the above-described upper limits.

For example, the lower limit of the ratio (M4/M5) of the mole number (M4) of the monomer unit of Formula 4 above to the mole number (M5) of the monomer unit of Formula 5 above in the conductive copolymer may be 1, 1.5, 2, 2.5, 3, 3.5, 4, 4.5, 5, 5.5, 6, 6.5, 7, 7.5, 8, or 8.5 or so, and the upper limit thereof may be 20, 19, 18, 17, 16, 15, 14, 13, 12, 11, 10, 9, 8, 7, 6, 5, or 4 or so. The ratio M4/M5 may be within a range of more than or equal to, or more than any one of the above-described lower limits; or within a range of less than or equal to, or less than any one of the above-described upper limits; or within a range of more than or equal to, or more than any one of the above-described lower limits while being less than or equal to, or less than any one of the above-described upper limits.

The lower limit of the ratio of the total mole number of the monomer units of Formulas 4 and 5 above in the conductive copolymer relative to the mole number of the total monomer units included in the copolymer may be 50 mol%, 55 mol%, 60 mol%, 65 mol%, 70 mol%, 75 mol%, 80 mol%, 85 mol%, 90 mol%, or 95 mol% or so, and the upper limit thereof may be 100 mol%, 95 mol%, or 90 mol% or so. The ratio may be within a range of more than or equal to, or more than any one of the above-described lower limits; or within a range of less than or equal to, or less than any one of the above-described upper limits; or within a range of more than or equal to, or more than any one of the above-described lower limits

while being less than or equal to, or less than any one of the above-described upper limits.

The conductive copolymer may further include other monomer units if it contains the above-mentioned units in the above ratio.

The polymer layer includes the conductive copolymer, and accordingly, it can exhibit the above-described properties. The polymer layer may also include any additional components if it includes the conductive copolymer.

The lower limit of the thickness of the polymer layer may be 10 nm, 50 nm, 100 nm, 150 nm, 200 nm, 250 nm, 300 nm, 350 nm, 400 nm, 450 nm, or 500 nm or so, and the upper limit thereof may be 2 μm, 1.5 μm, 1 μm, 950 nm, 900 nm, 850 nm, 800 nm, 750 nm, 700 nm, 650 nm, 600 nm, 550 nm, 500 nm, 450 nm, 400 nm, 350 nm, or 300 nm or so. The thickness may be within a range of more than or equal to, or more than any one of the above-described lower limits; or within a range of less than or equal to, or less than any one of the above-described upper limits; or within a range of more than or equal to, or more than any one of the above-described lower limits while being less than or equal to, or less than any one of the above-described upper limits.

The present application also relates to a method for manufacturing the electrode current collector. The manufacturing method comprises a process for controlling the crystallinity of the conductive copolymer for the desired PCT effect.

The manufacturing method may comprise a step of forming a polymer layer using a polymer solution containing the conductive copolymer.

In the step, the detailed description of the conductive copolymer used is as described above, and the coating liquid can be prepared by dissolving the copolymer in an appropriate solvent. At this time, if the solvent can dissolve at least part of the conductive copolymer, the type thereof is not particularly limited.

In the step, the lower limit of the concentration of the conductive copolymer present in the polymer solution may be 0.5 weight%, 1 weight%, 1.5 weight%, 2 weight%, 2.5 weight%, or 3 weight% or so, and the upper limit thereof may be 20 weight%, 18 weight%, 16 weight%, 14 weight%, 12 weight%, 10 weight%, 9 weight%, 8 weight%, 7 weight%, 6 weight%, 5 weight%, 4 weight%, or 3 weight% or so. The ratio may be within a range of more than or equal to, or more than any one of the above-described lower limits; or within a range of less than or equal to, or less than any one of the above-described upper limits; or within a range of more than or equal to, or more than any one of the above-described lower limits while being less than or equal to, or less than any one of the above-described upper limits. Such a concentration may be changed as needed.

The conductive copolymer can be formed by a known polymerization method. For example, a method using an oxidation polymerization reaction or a method using a radical reaction are representatively known as a method for producing polythiophene.

A polymer layer is formed on the current collector main body using the prepared polymer solution. This process usually comprises steps of coating the polymer solution on the current collector main body, and annealing the coated coating liquid. In this process, the crystallinity of the conductive copolymer can also be controlled by the annealing conditions.

For example, the temperature T and/or time H of the annealing can be adjusted.

For example, the lower limit of the temperature T may be 50°C, 55°C, 60°C, 65°C, 70°C, 75°C, 80°C, 85°C, 90°C, 95°C, 100°C, 105°C, 110°C, 115°C, 120°C, or 125°C or so, and the upper limit thereof may be 200°C, 195°C, 190°C, 185°C, 180°C, 175°C, 170°C, 165°C, 160°C, 155°C, 150°C, 145°C, 140°C, 135°C, 130°C, 125°C, 120°C, 115°C, 110°C, 105°C, 100°C, 95°C, or 90°C or so. The temperature may be within a range of less than or equal to, or less than any one of the above-described upper limits; within a range of more than or equal to, or more than any one of the above-described lower limits; or within a range of more than or equal to, or more than any one of the above-described lower limits while being less than or equal to, or less than any one of the above-described upper limits. Within this range, the alignment state of the long-chain hydrocarbon functional groups in the conductive copolymer can be appropriately adjusted, and accordingly, it is possible to secure the desired crystallinity.

To achieve the purpose, the product (T×H) of the annealing temperature T and time H can be adjusted. For example, the lower limit of the product (T×H) of the annealing temperature T and time H may be 10°C·hour, 15°C·hour, 20°C·hour, 25°C·hour, 30°C·hour, 35°C·hour, 40°C·hour, 45°C·hour, 50°C·hour, 75°C·hour, 100°C·hour, 110°C·hour, 120°C·hour, 130°C·hour, 150°C·hour, 160°C·hour, 170°C·hour, 180°C·hour, 190°C·hour, 200°C·hour, 210°C·hour, 220°C·hour, or 230°C·hour or so, and the upper limit thereof may be 100000°C·hour, 95000°C·hour, 90000°C·hour, 85000°C·hour, 80000°C·hour, 75000°C·hour, 70000°C·hour, 65000°C·hour, 60000°C·hour, 55000°C·hour, 50000°C·hour, 45000°C·hour, 40000°C·hour, 35000°C·hour, 30000°C·hour, 25000°C·hour, 20000°C·hour, 15000°C·hour, 10000°C·hour, 9500°C·hour, 9000°C·hour, 8500°C·hour, 8000°C·hour, 7500°C·hour, 7000°C·hour, 6500°C·hour, 6000°C·hour, 5500°C·hour, 5000°C·hour, 4500°C·hour, 4000°C·hour, 3500°C·hour, 3000°C·hour, 2500°C·hour, 2000°C·hour, 1500°C·hour, 1400°C·hour, 1300°C·hour, 1200°C·hour, 1100°C·hour, 1000°C·hour, 900°C·hour, 800°C·hour, 700°C·hour, 600°C·hour, 500°C·hour, 400°C·hour, 300°C·hour, 200°C·hour, 100°C·hour, 90°C·hour, 80°C·hour, 70°C·hour, 60°C·hour, 50°C·hour, 45°C·hour, or 40°C·hour or so. The product (T×H) may be within a range of less than or equal to, or less than any one of the above-described upper limits; within a range of more than

or equal to, or more than any one of the above-described lower limits; or within a range of more than or equal to, or more than any one of the above-described lower limits while being less than or equal to, or less than any one of the above-described upper limits. Within this range, the alignment state of the long-chain hydrocarbon functional groups in the conductive copolymer can be appropriately adjusted, and accordingly, it is possible to secure the desired crystallinity.

The method of coating the coating liquid is not particularly limited, and any known coating method may be applied.

In the present application, the desired polymer layer, and the current collector comprising the same are manufactured through the above process. The above process may comprise appropriate post-treatment processes if necessary.

The present application also relates to an electrode comprising the current collector. The electrode may comprise an active material layer formed on the polymer layer of the current collector.

A commonly applied layer can also be used as the active material layer.

Typically, the active material layer comprises an electrode active material. The specific type of the electrode active material is not particularly limited, and materials to form a positive electrode or a negative electrode can usually be used.

For example, when the active material layer is a positive electrode active material layer, the electrode active material may include layered compounds such as lithium cobalt oxide ($LiCoO_2$) or lithium nickel oxide ($LiNiO_2$), or compounds substituted with one or more transition metals; lithium iron oxides such as $LiFe_3O_4$; lithium manganese oxides such as Formula $Li_{1+c1}Mn_{2-c1}O_4$ ($0 \leq c1 \leq 0.33$), $LiMnO_3$, $LiMn_2O_3$, or $LiMnO_2$; lithium copper oxides ($Li_2CuO_2$); vanadium oxides such as $LiV_3O_8$, $V_2O_5$, or $Cu_2V_2O_7$; Ni-site lithium nickel oxides represented by Formula $LiNi_{1-c2}M_{c2}O_2$ (where M is at least one selected from the group consisting of Co, Mn, Al, Cu, Fe, Mg, B and Ga, and $0.01 \leq c2 \leq 0.3$ is satisfied); lithium manganese composite oxides represented by Formula $LiMn_{2-c3}M_{c3}O_2$ (where M is at least one selected from the group consisting of Co, Ni, Fe, Cr, Zn, and Ta, and $0.01 \leq c3 \leq 0.1$ is satisfied) or $Li_2Mn_3MO_8$ (here, M is at least one selected from the group consisting of Fe, Co, Ni, Cu and Zn); lithium nickel cobalt manganese (NCM) composite oxides, lithium nickel cobalt manganese aluminum (NCMA) composite oxides, and $LiMn_2O_4$ in which a part of Li in the formula is substituted with alkaline earth metal ions, and the like, but is not limited thereto.

When the active material layer is a negative electrode active material layer, as the electrode active material, for example, a compound capable of reversible intercalation and deintercalation of lithium may be used. A specific example may include carbonaceous materials such as artificial graphite, natural graphite, graphitized carbon fiber, and amorphous carbon; metallic compounds capable of alloying with lithium, such as Si, Al, Sn, Pb, Zn, Bi, In, Mg, Ga, Cd, Si alloy, Sn alloy, or Al alloy; metal oxides capable of doping and de-doping lithium, such as $SiO_a$ ($0 < a < 2$), $SnO_2$, vanadium oxide, and lithium vanadium oxide; or a composite comprising the metallic compound and the carbonaceous materials such as a Si-C composite or a Sn-C composite, and the like, and any one or a mixture of two or more of the foregoing may be used.

A metal lithium thin film may also be used as the negative electrode active material, and as the carbon material, low crystalline carbon and high crystalline carbon, and the like may also be used. As the low crystalline carbon, soft carbon and hard carbon are representative, and as the high crystalline carbon, high-temperature baked carbons such as amorphous, plate-like, scale-like, spherical, or fibrous natural graphite or artificial graphite, Kish graphite, pyrolytic carbon, mesophase pitch based carbon fiber, mesocarbon microbeads, mesophase pitches, and petroleum and coal tar pitch derived cokes are representative.

The electrode active material may be included in the active material layer within a range of about 80 weight% to 99.5 weight%, or within a range of 88 weight% to 99 weight% relative to the total weight of the active material layer, but the ratio may be changed depending on the use or design of the electrode.

The active material layer may further comprise a binder. The binder serves to improve attachment between the active materials, and adhesive force on between the active material layer and the current collector main body. An example of the binder is not particularly limited, and one or more may be selected from the group consisting of, for example, PVDF (poly(vinylidene fluoride), PVA (poly(vinyl alcohol)), SBR (styrene butadiene rubber), PEO (poly(ethylene oxide)), CMC (carboxyl methyl cellulose), cellulose acetate, cellulose acetate butylate, cellulose acetate propionate, cyanoethylpullulan, cyanoethyl polyvinyl alcohol, cyanoethyl cellulose, cyanoethyl sucrose, pullulan, polymethylmethacrylate, polybutylacrylate, polyacrylonitrile, polyvinylpyrrolidone, polyvinyl acetate, polyethylene-co-vinyl acetate, and polyarylate, and used.

In one example, the binder may be included in the active material layer within a range of 0.1 parts by weight to 10 parts by weight, or 0.5 parts by weight to 5 parts by weight relative to 100 parts by weight of the electrode active material, but is not limited thereto.

The active material layer may further comprise a conductive material, as needed. If the conductive material has conductivity and doesn't cause chemical changes in the secondary battery, it is not particularly limited, any known material can be used. For example, graphite such as natural graphite or artificial graphite; carbon black, such as carbon black, acetylene black, Ketjen black, channel black, furnace black, lamp black, or thermal black; conductive fibers such as carbon fibers or metal fibers; conductive tubes such as carbon nanotubes (CNTs); fluorocarbon, metal powders such as aluminum, or nickel powder; conductive whiskers such as zinc oxide or potassium titanate; conductive metal oxides

such as titanium oxide; conductive materials such as polyphenylene derivatives, and the like may be used.

In one example, the conductive material may be included in the active material layer in an amount of 0.1 parts by weight to 20 parts by weight, or 0.3 parts by weight to 10 parts by weight relative to 100 parts by weight of the electrode active material, but is not limited thereto.

The active material layer may also further comprise any necessary known components in addition to the above-described components optionally.

The present application also relates to a method for manufacturing the electrode.

Such a manufacturing method of the present application may comprise a step of forming the active material layer on the polymer layer on the current collector main body.

Also, the method of forming the active material layer on the polymer layer is not particularly limited. Typically, the active material layer is formed by coating a slurry containing the electrode active material, binder, and conductive material, and the like on the current collector main body (on the polymer layer), followed by drying, and then rolling, where such a known method can also be equally applied to the present application.

The present application also relates to an electrode assembly or electrochemical element comprising such an electrode, for example, a secondary battery.

The electrochemical element may comprise the electrode as the positive electrode and/or the negative electrode. If the electrode of the present application is used as the positive electrode and/or the negative electrode, other configurations or manufacturing methods of the electrochemical element are not particularly limited, and known methods can be applied.

## Advantageous Effects

The present application relates to a current collector, and a use thereof. The present application can provide a current collector capable of forming an electrode, which does not affect performance and operation of a secondary battery by exhibiting, in a normal state, excellent electrical characteristics including low resistance, and can ensure stability by blocking, in an abnormal state, energization of an electrode assembly through resistance increase, and a use thereof.

## Description of Drawings

Figure 1 is a cross-sectional diagram of an exemplary current collector of the present application.

Figure 2 is a cross-sectional diagram of an exemplary electrode of the present application.

Figure 3 is the NMR analysis results of the monomer of Preparation Example 1.

Figure 4 is the NMR analysis results of the monomer of Preparation Example 2.

## Mode for Invention

Hereinafter, the contents of the present application will be described in detail through examples and comparative examples, but the scope of the present application is not limited by the following examples.

### 1. NMR analysis method

$^1$H-NMR analyses were performed at room temperature using an NMR spectrometer including a Bruker UltraShield spectrometer (300 MHz) with a 5 mm triple resonance probe. A sample was diluted in a solvent ($CDCl_3$) for NMR measurement to a concentration of about 10 mg/ml or so and used, and the chemical shift was expressed in ppm.

### 2. GPC (Gel Permeation Chromatograph)

Molecular weight characteristics were measured using GPC (Gel permeation chromatography). A sample is placed in a 5 mL vial and diluted with chloroform to a concentration of about 1 mg/mL or so. The standard sample for calibration and the sample to be analyzed were filtered through a syringe filter (pore size: 0.45 μm), and then molecular weight characteristics were measured. As the analysis program, Waters' Empower 3 was used, and a weight average molecular weight (Mw) and a number average molecular weight (Mn) were each obtained by comparing the elution time of the sample with the calibration curve, and the molecular weight distribution (PDI) was calculated by the ratio (Mw/Mn).

The measurement conditions of GPC are as follows.

<GPC measurement conditions>

Device: Waters' 2414

Column: using 3 Styragels from Waters

Solvent: THF (tetrahydrofuran)

Column temperature: 35°C

Sample concentration: 1 mg/mL, 1 μL injection

Standard sample: polystyrene (Mp: 3900000, 723000, 316500, 52200, 31400, 7200, 3940, 485)

### 3. Thickness measurement

The polymer layer or the like was subjected to cross-section using ion milling equipment (Hitachi, IM5000), and then the thickness was measured by photographing an image with a SEM (Scanning Electron Microscope) (JEOL, JSM-7200F).

The conditions for forming the cross section by the ion milling were performed by setting the equipment in cross-section milling mode, the speed (reciprocation/min) of 3, the acceleration voltage of 6.0 kV, the discharge voltage of 15 kV, the current of 150 μA, and the time of 4 hours.

### 4. Oxidation potential measurement method

An oxidation potential was measured in the following manner. A polymer layer was formed to a thickness of about 10 μm or so using a conductive copolymer on an aluminum foil (Al Foil) with a thickness of about 15 μm. The polymer layer was formed in the same manner as described in each of examples or comparative examples, and was formed to have a thickness of 10 μm or so. A separator and a lithium film were laminated on the polymer layer to prepare a laminate in which the aluminum foil/polymer layer/separator/lithium film were laminated, and the laminate was punched into a circle with a diameter of about 1.4 cm. A coin cell was manufactured using the circularly punched laminate and an electrolyte (using Welcos CR2032 coin cell kit). As the separator, the WL20C model from W Scope Korea was used; as the lithium film, a lithium film with a thickness of about 100 μm was used; and as the electrolyte, Enchem's product (1M $LiPF_6$ solution (solvent: EC/DMC/EMC =3/4/3 (mass ratio), EC: Ethylene Carbonate, DMC: dimethyl carbonate, EMC: ethylmethyl carbonate)) was used.

The oxidation potential of the coin cell was measured using an electrochemical meter (potentiostat) (Princeton Applied Research, PARASTAT-MC) at 25°C. The oxidation potential was measured by measuring CV (Cyclic Voltammetry) in the range of 1.5V to 5.5V at a scan rate of 0.17 mV/sec to 0.5 mV/sec.

### 5. DC resistance measurement method

DC resistance was evaluated using the same coin cell as used in the oxidation potential measurement. A voltage of 4.3eV was applied to the coin cell for 10 minutes at room temperature (25°C), and the DC resistance was measured using a Fluke digital multi-tester (FLUKE-87-5).

### 6. AC impedance resistance

AC impedance resistance was evaluated through EIS (Electrochemical Impedance Spectronization) using the same coin cell as used for oxidation potential measurement. A voltage of 4.3V was applied to the coin cell for 10 minutes at room temperature (25°C), Nyquist plot was obtained by an EIS measurement method at 50,000 Hz to 0.1 Hz, and the AC impedance resistance obtained in the high frequency region was measured. An electrochemical meter (potentiostat) (Princeton Applied Research, PARASTAT-MC) was used as the EIS measurement device.

**7. Measurement of maximum resistance change rate (DC resistance)**

The maximum resistance change rate ΔR1 is determined according to Equation 1 below.

<Equation 1>

$$\Delta R1 = Max\{(R_{n+5}/R_n)/5\}$$

In Equation 1, $R_n$ is the DC resistance at any temperature n°C within a range of 25°C to 135°C, and $R_{n+5}$ is the DC resistance at a temperature ((n+5)°C) 5°C higher than the temperature n°C.
The ΔR1 is measured in the following manner.
A coin cell for DC resistance measurement is placed in the center of a convection oven (JEIO TECH, OF3-05W), and the temperature of the oven is set to an initial temperature of 25°C, a final temperature of 135°C, and a temperature increase of 5°C per minute. The coin cell is connected to a resistance measurement multimeter (Fluke's digital multi-tester (FLUKE-87-5)) outside the oven to enable the resistance measurement. Subsequently, in a state where the temperature increases as set, the DC resistance is measured at each temperature (measured up to 135°C while increasing the measurement temperature by 5°C in the order of 25°C, 30°C, 35°C, and 40°C). $R_n$ and $R_{n+5}$ in Equation 1 for each measurement temperature are each measured, $R_{n+5}/R_n$ ($R_{30}/R_{25}$, $R_{35}/R_{30}$ ~ $R_{135}/R_{130}$) are calculated, and then divided by 5.
After obtaining $(R_{n+5}/R_n)/5$ in the temperature range of 25°C to 135°C, the maximum value thereof is obtained as the ΔR1. Through the ΔR1, the temperature responsiveness of the resistance increase of the conductive copolymer (polymer layer) at an onset temperature can be identified.
The onset temperature is the temperature n°C where $(R_{n+5}/R_n)/5$ shows the maximum value.
As the coin cell for DC resistance measurement, the same one applied upon oxidation potential measurement was used.

**8. Measurement of maximum resistance change rate (AC impedance)**

The maximum resistance change rate ΔR2 is determined according to Equation 2 below.

<Equation 2>

$$\Delta R2 = Max\{(R_{z+5}/R_z)/5\}$$

In Equation 2, $R_z$ is the AC impedance resistance at any temperature n°C within a range of 25°C to 135°C, and $R_{z+5}$ is the AC impedance resistance at a temperature ((n+5)°C) 5°C higher than the temperature n°C.
The ΔR2 is measured in the following manner.
A coin cell for AC impedance resistance measurement is placed in the center of a convection oven (JEIO TECH, OF3-05W), and the temperature of the oven is set to an initial temperature of 25°C, a final temperature of 135°C, and a temperature increase of 5°C per minute. The coin cell is connected to a resistance meter (potentiostat) (Princeton Applied Research, PARASTAT-MC) outside the oven to enable the resistance measurement. Subsequently, in a state where the temperature increases as set, the AC impedance resistance is measured at each temperature (measured up to 135°C while increasing the measurement temperature by 5°C in the order of 25°C, 30°C, 35°C, and 40°C). $R_z$ and $R_{z+5}$ in Equation 2 for each measurement temperature are each measured, $R_{z+5}/R_z$ ($R_{30}/R_{25}$, $R_{35}/R_{30}$ ~ $R_{135}/R_{130}$) are calculated, and then divided by 5.
After obtaining $(R_{z+5}/R_z)/5$ in the temperature range of 25°C to 135°C, the maximum value thereof is obtained as the ΔR2.
Through the ΔR2, the temperature responsiveness of the resistance increase of the conductive copolymer (polymer layer) at an onset temperature can be identified.
The onset temperature is the temperature n°C where $(R_{z+5}/R_z)/5$ shows the maximum value.
As the coin cell for measurement, the same one applied upon oxidation potential measurement was used.
The AC impedance resistance was determined as the resistance obtained from the semicircle in the high frequency region by applying a voltage of 4.3V for 10 minutes, and obtaining a Nyquist plot using the EIS measurement method at 50,000 Hz to 0.1 Hz.

### 9. Discharged capacity measurement

A discharged capacity for identifying the results of Equation 3 below was evaluated in the following manner.

<Equation 3>

$$\Delta R3 = 100 \times (C_1 - C_2)/C_1$$

In Equation 3, ΔR3 is the change rate (%) of the discharged capacity, $C_1$ is a discharged capacity at room temperature (about 25°C), and $C_2$ is a discharged capacity after storage at 70°C for 60 hours.

A coin cell (standard capacity: 200 mAh/g) for identifying the discharged capacity of Equation 3 was produced using a CR2032 standard coin cell kit (Welkos CR2032 coin cell kit). The electrode prepared in each of Examples or Comparative Examples was used as a positive electrode, and a lithium film (thickness: 100 μm) was used as a negative electrode. As the electrolyte of a carbonate-based electrolyte, a 1M $LiPF_6$ solution (solvent: EC/DMC/EMC=3/4/3 (mass ratio), EC: ethylene carbonate, DMC: dimethyl carbonate, EMC: ethylmethyl carbonate) was used, and a PE (poly(ethylene)) separator (WL20C model from W Scope Korea) was used as a separator.

The coin cell was charged/discharged once at 25°C, and the capacity at 0.2C was set as the discharged capacity in Equation 3 above. The one charge/discharge means that a process of charging it in a CC (constant current)/CV (constant voltage) method at a rate of 0.2C by setting a final charging voltage of 4.5V and a final charging current of 1mA, and discharging it in a CC (constant current) method at a rate of 0.2C by setting a final discharging voltage of 3.0V is repeated once as one cycle. The discharged capacity after one charge/discharge was applied as the discharged capacity ($C_1$, $C_2$) in Equation 3 above.

Immediately after manufacturing the coin cell, the above measurement method was applied to obtain $C_1$, and then after storing the coin cell at 70°C for 60 hours, the above measurement method was applied to obtain $C_2$.

### 10. Discharged capacity measurement

A discharged capacity for identifying the results of Equation 4 below was evaluated in the following manner.

<Equation 4>

$$\Delta R4 = 100 \times (C_1 - C_3)/C_1$$

In Equation 4, ΔR4 is the change rate (%) of the discharged capacity, $C_1$ is a discharged capacity at room temperature (about 25°C), and $C_3$ is a discharged capacity after storage at 130°C for 10 minutes.

A coin cell for identifying the discharged capacity of Equation 4 was manufactured in the same manner as in the coin cell for identifying Equation 3, and the discharged capacity measurement was also performed in the same manner.

That is, immediately after manufacturing the coin cell, it is applied to the measurement method for identifying Equation 3 above to obtain $C_1$.

Thereafter, after storing the coin cell at 70°C for 60 hours and then storing it again at 130°C for 10 minutes, $C_3$ was obtained.

$C_3$ was obtained in the following manner. As a process of charging it in a CC (constant current)/CV (constant voltage) method at a rate of 0.5C by setting a final charging voltage of 4.5V and a final charging current of 1mA, and discharging it in a CC (constant current) method at a rate of 2C by setting a final discharging voltage of 3.0V again is set to one cycle, the cycle was repeated 30 times, and the discharged capacity after 30 charge/discharge cycles was applied as $C_3$ in Equation 4 above. The 30 charge/discharge cycles were performed at 45°C.

### Preparation Example 1. Synthesis of monomer (A)

A monomer of Formula A below (monomer (A)) (3,4-[3,3'-dibutylpropylenedioxy]thiophene) was synthesized in the following manner.

[Formula A]

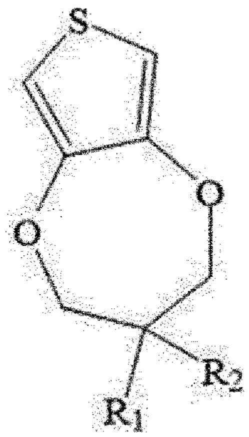

In Formula A, $R_1$ and $R_2$ are linear butyl groups.

In 200 ml of toluene, 6 g (41.61 mmol, 1 eq) of 3,4-dimethoxythiophene and 10.187 g (54.10 mmol, 1.3 eq) of 2,2-dibutyl-1,3-propanediol were dissolved together with 500 mg of p-toluenesulfonic acid (p- TsOH), and mixed. While the mixture was refluxed at 120°C, methanol produced during transetherification was removed using filled 4A type molecular sieves with a Soxhlet extractor. The reactant was refluxed for 24 hours, and then quenched with water, and extracted with ethyl acetate, and then washed with brine, and dried over magnesium sulfate ($MgSO_4$). The solvent was removed using a rotary evaporator, and the residue was purified by column chromatography eluting with methylene chloride/hexane (1:4) to obtain the compound 1 (3,4-(3,3'-dibutylpropylenedioxy)thiophene). The NMR analysis results for the target compound (monomer (A)) are as shown in Figure 3.

**Preparation Example 2. Synthesis of monomer (B)**

A monomer of Formula B below was synthesized in the following manner.

[Formula B]

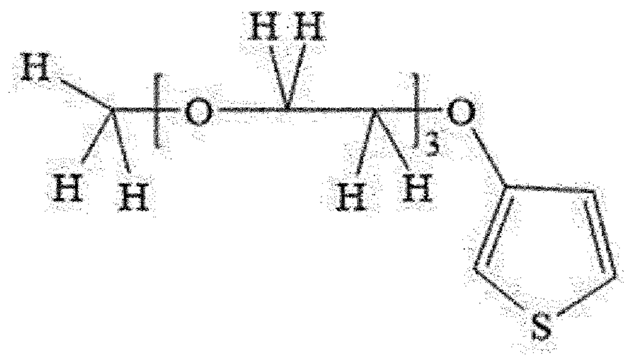

In 100 ml of toluene, 1.372 g (12.02 mmol, 1eq) of 3-methoxythiophene and 3 g (16.83 mmol, 1.4eq) of triethylene glycol monomethyl ether were dissolved together with 230 mg of p-toluenesulfonic acid (p-TsOH), and mixed. While the mixture was refluxed at 120°C, methanol produced by transetherification was removed using filled 4A type molecular sieves with a Soxhlet extractor. The reactant was refluxed for 24 hours, and then quenched with water, and extracted with ethyl acetate, and then washed with brine, and dried over magnesium sulfate ($MgSO_4$). The solvent was removed using a rotary evaporator, and the residue was purified by column chromatography eluting with methylene chloride/hexane (2:1) to obtain the target compound (monomer (B)). The NMR analysis results for the target compound (monomer (B)) are as shown in Figure 4.

**Example 1.**

**Synthesis of polythiophene (A)**

To a solution in which 3.20 g (19.71 mmol, 3eq) of iron (III) chloride was dissolved in 150ml of methylene chloride, 1.58 g (5.91 mmol, 0.9 eq) of the monomer (A) of Preparation Example 1 and 0.16 g (0.66 mmol, 0.1 eq) of the monomer (B) of Preparation Example 2 were introduced and polymerized at 25°C for 24 hours to prepare polythiophene (A).

The polymerization solution was placed in an osmosis membrane with an MWCO (molecular weight of cut-off) of 5000, and then immersed in 200ml of an acetonitrile solvent to remove the unreacted iron (III) chloride, monomers, and low molecular weight oligomer. The residue precipitated inside the osmosis membrane was washed with methanol, and dried at 60°C for 12 hours to prepare polythiophene (A).

The weight average molecular weight (Mw) and number average molecular weight (Mn) of polythiophene (A) were 60,200 g/mol and 18,500 g/mol, respectively, and the oxidation potential was about 3.5 V or so.

**Manufacturing of electrode**

As a current collector main body, an Al foil with a thickness of about 15 μm or so was used. A coating liquid was prepared by dispersing the polythiophene (A) in a solvent (chloroform) at a concentration of 2 weight% or so. The coating liquid was coated on the current collector main body using a bar coating method, and dried and annealed at 130°C for 1 hour or so to form a polymer layer with a thickness of about 300 nm. Subsequently, an active material layer was formed on the polymer layer. A slurry comprising lithium cobalt oxide ($LiCoO_2$), a carbon-based conductive material (ECP (Ketjen Black) 0.5%, SFG (Trimrex graphite) 0.4%, DB (Denka Black) 0.4%), PVDF (polyvinylidene fluoride), and NMP (N-methyl-2-pyrrolidone) in a weight ratio of 75:1:1:23 ($LiCoO_2$: conductive material: PVDF: NMP) was applied on the polymer layer to a thickness of about 90 μm or so using a doctor blade, and further dried under a vacuum condition at 120°C after drying at room temperature (about 25°C) to prepare the active material layer. Subsequently, an electrode was manufactured by rolling it to a porosity of about 25% or so.

**Example 2.**

An electrode was manufactured in the same manner as in Example 1, except that the polymer layer was formed to a thickness of about 500 nm or so.

**Example 3.**

**Synthesis of polythiophene (C)**

To a solution in which 3.20 g (19.71 mmol, 3eq) of iron (III) chloride was dissolved in 150ml of methylene chloride, 1.41 g (5.26 mmol, 0.8 eq) of the monomer (A) of Preparation Example 1 and 0.35 g (1.32 mmol, 0.2 eq) of the monomer (B) of Preparation Example 2 were introduced and polymerized at 25°C for 24 hours to prepare polythiophene (C). In the polythiophene (C), the weight ratio (A: B) of the monomer (A) unit of Preparation Example 1 and the monomer (B) unit is about 4:1.

The polymerization solution was placed in an osmosis membrane with an MWCO (molecular weight of cut-off) of 5000, and then immersed in 200ml of an acetonitrile solvent to remove components such as the unreacted iron (III) chloride, and monomers. The residue precipitated inside the osmosis membrane was washed with methanol, and dried at 60°C for 12 hours to prepare polythiophene (C).

The weight average molecular weight (Mw) and number average molecular weight (Mn) of polythiophene (C) were 48,300 g/mol and 11,200 g/mol, respectively, and the oxidation potential was about 3.55 V or so.

**Manufacturing of electrode**

An electrode was manufactured in the same manner as in Example 1, where the thickness of the polymer layer was about 300 nm.

**Example 4.**

**Synthesis of polythiophene (B)**

To a solution in which 3.20 g (19.71 mmol, 3eq) of iron (III) chloride was dissolved in 150ml of methylene chloride, 1.16 g (5.9 mmol, 0.9 eq) of 3-octylthiophene and 0.16 g (0.66 mmol, 0.1 eq) of the monomer (B) of Preparation Example 2 were introduced and polymerized at 25°C for 24 hours to prepare polythiophene (B). In the polythiophene (B), the weight

ratio (3-OT: B) of the prepared 3-octylthiophene unit (3-OT) and the monomer (B) of Preparation Example 2 is about 7:1.

The polymerization solution was placed in an osmosis membrane with an MWCO (molecular weight of cut-off) of 5000, and then immersed in 200ml of an acetonitrile solvent to remove the unreacted iron (III) chloride, and monomers. The residue precipitated inside the osmosis membrane was washed with methanol, and dried at 60°C for 12 hours to prepare polythiophene (B).

The weight average molecular weight (Mw) and number average molecular weight (Mn) of polythiophene (B) were 56,500 g/mol and 12,800 g/mol, respectively, and the oxidation potential was about 3.7 V or so.

**Manufacturing of electrode**

An electrode was manufactured in the same manner as in Example 1, where the thickness of the polymer layer was about 300 nm.

**Example 5.**

**Synthesis of polythiophene (D)**

To a solution in which 3.20 g (19.71 mmol, 3eq) of iron (III) chloride was dissolved in 150ml of methylene chloride, 1.03 g (5.24 mmol, 0.8 eq) of 3-octyl thiophene and 0.32 g (1.32 mmol, 0.2 eq) of the monomer (B) of Preparation Example 2 were introduced and polymerized at 25°C for 24 hours to prepare polythiophene (D). In the polythiophene (D), the weight ratio (3-OT: B) of 3-octylthiophene unit (3-OT) to the monomer (B) is about 3:1.

The polymerization solution was placed in an osmosis membrane with an MWCO (molecular weight of cut-off) of 5000, and then immersed in 200ml of an acetonitrile solvent to remove the unreacted iron (III) chloride, and monomers. The residue precipitated inside the osmosis membrane was washed with methanol, and dried at 60°C for 12 hours to prepare polythiophene (D).

The weight average molecular weight (Mw) and number average molecular weight (Mn) of polythiophene (D) were 45,700 g/mol and 10,300 g/mol, respectively, and the oxidation potential was about 3.7 V or so.

**Manufacturing of electrode**

An electrode was manufactured in the same manner as in Example 1, where the thickness of the polymer layer was about 500 nm.

**Example 6.**

An electrode was manufactured in the same manner as in Example 1. In Example 6, when forming a polymer layer, it was formed by coating the coating liquid on the current collector main body using a bar coating method and drying it at 90°C for 20 minutes or so.

**Comparative Example 1.**

An electrode was manufactured in the same manner as in Example 1, except that the polymer layer was not formed.

**Comparative Example 2.**

**Synthesis of polythiophene (F)**

To a solution in which 3.20 g (19.71 mmol, 3eq) of iron (III) chloride was dissolved in 150ml of methylene chloride, 3-octylthiophene (6.56 mmol, 1.29 eq) was introduced and polymerized at 25°C for 24 hours to prepare polythiophene (F).

The polymerization solution was placed in an osmosis membrane with an MWCO (molecular weight of cut-off) of 5000, and then immersed in 200ml of an acetonitrile solvent to remove the unreacted iron (III) chloride, and monomers. The residue precipitated inside the osmosis membrane was washed with methanol, and dried at 60°C for 12 hours to prepare polythiophene (F).

The weight average molecular weight (Mw) and number average molecular weight (Mn) of polythiophene (F) were 59,200 g/mol and 17,400 g/mol, respectively, and the oxidation potential was about 3.7 V or so.

**Manufacturing of electrode**

An electrode was manufactured in the same manner as in Example 1, where the thickness of the polymer layer was about 300 nm.

The measurement results for the manufactured electrodes are summarized in Tables 1 and 2 below. In Tables 1 and 2 below, $C_1$ in Equation 3 and $C_1$ in Equation 4 should theoretically represent the same value, but in actual experiments, some differences occurred within the error range.

[Table 1]

| | | Example | | | | | |
|---|---|---|---|---|---|---|---|
| | | 1 | 2 | 3 | 4 | 5 | 6 |
| DC resistance (Ωcm) | | 350 | 608 | 432 | 412 | 489 | 530 |
| AC impedance (Ω) | | 48 | 72 | 57 | 66 | 89 | 60 |
| Equation 1 | ΔR1 | 208.3 | 277.2 | 179.8 | 412.5 | 354.2 | 55 |
| | Onset (°C) | 90 | 90 | 95 | 90 | 95 | 85 |
| Equation 2 | ΔR2 | 18.8 | 23.3 | 15.4 | 33.2 | 27.3 | 8.2 |
| | Onset (°C) | 90 | 90 | 95 | 90 | 95 | 85 |
| Equation 3 | C1 | 198.3 | 193.3 | 199.2 | 195.4 | 196.9 | 199.6 |
| | C2 | 195.5 | 189.8 | 197.8 | 192.2 | 194.7 | 195.7 |
| | ΔR3 | 1.4 | 1.8 | 0.7 | 1.6 | 1.1 | 2.0 |
| Equation 4 | C1 | 198.1 | 193.5 | 198.7 | 196.2 | 199.8 | 199.5 |
| | C3 | 65.2 | 45.5 | 78.9 | 61.3 | 80.8 | 168.2 |
| | ΔR4 | 67.1 | 76.5 | 60.3 | 68.8 | 59.6 | 15.7 |

[Table 2]

| | | Comparative Example | |
|---|---|---|---|
| | | 1 | 2 |
| DC resistance (Ωcm) | | 5.3 | 335.2 |
| AC impedance (Ω) | | 2.1 | 45 |
| Equation 1 | ΔR1 | 1.2 | 253 |
| | Onset (°C) | - | 80 |
| Equation 2 | ΔΔR2 | 2.5 | 33.4 |
| | Onset (°C) | - | 75 |
| Equation 3 | C1 | 199.8 | 198.7 |
| | C2 | 198.5 | 133.7 |
| | ΔR3 | 0.7 | 32.7 |
| Equation 4 | C1 | 199.2 | 197.8 |
| | C3 | 165.4 | 56.7 |
| | ΔR4 | 17 | 71.3 |

Through Tables 1 and 2, in the case of the electrodes according to the present application, it can be confirmed that they show low resistance in the normal state, thereby doing not affect the performance and operation of the secondary battery, and show characteristics capable of ensuring stability by blocking energization of the electrode assembly through the resistance increase in the abnormal state caused by overcharging, high-temperature exposure, or outer shocks, and the like.

In addition, through comparison of Examples 1 and 6, it can be confirmed that even when the same type of conductive copolymer is used, the differences in effectiveness occur due to crystallinity changes depending on annealing conditions.

In the case of Comparative Example 1 without any polymer layer, the resistance increase did not occur even under high temperature conditions. In the case of Comparative Example 2, the resistance increase occurred under high temperature conditions, but because the onset temperature was low, it shows the resistance increase even when stored under high temperature conditions, thereby adversely affecting the performance of the battery.

## Claims

**1.** A current collector, comprising:

a current collector main body; and
a polymer layer on one or both sides of the current collector main body,
wherein the polymer layer comprises a conductive copolymer.

**2.** The current collector according to claim 1, wherein a DC resistance of the current collector is 10,000 Ω·cm or less at 25°C.

**3.** The current collector according to claim 1, wherein an AC impedance resistance of the current collector is 1,000Q or less.

**4.** The current collector according to claim 1, wherein a ΔR1 value according to Equation 1 below is 100 Ω·cm/°C or more:

[Equation 1]

$$\Delta R1 = \mathrm{Max}\{(R_{n+5}/R_n)/5\}$$

wherein, the $R_n$ is a DC resistance at any temperature n°C within a range of 25°C to 135°C, and the $R_{n+5}$ is a DC resistance at a temperature ((n+5)°C) 5°C higher than the temperature n°C, and the Max{($R_{n+5}/R_n$)/5} is the maximum value among the ($R_{n+5}/R_n$)/5 values confirmed within the temperature range of 25°C to 135°C.

**5.** The current collector according to claim 4, wherein the temperature of $R_n$ at which the ΔR1 is identified is more than 80°C.

**6.** The current collector according to claim 1, wherein a ΔR2 value according to Equation 2 below is 10Ω/°C or more:

[Equation 2]

$$\Delta R2 = \mathrm{Max}\{(R_{z+5}/R_z)/5\}$$

wherein, the $R_z$ is an AC impedance resistance at any temperature n°C within a range of 25°C to 135°C, and the $R_{z+5}$ is an AC impedance resistance at a temperature ((n+5)°C) 5°C higher than the temperature n°C, and the Max {($R_{z+5}/R_z$)/5} is the maximum value among the ($R_{z+5}/R_z$)/5 values confirmed within the temperature range of 25°C to 135°C.

**7.** The current collector according to claim 6, wherein the temperature of $R_z$ at which the ΔR2 is identified is 80°C or more.

**8.** The current collector according to claim 1, wherein an absolute value of a ΔR3 value according to Equation 3 is less than 10%:

[Equation 3]

$$\Delta R3 = 100 \times (C_1\text{-}C_2)/C_1$$

wherein, the $C_1$ is a discharged capacity at 25°C, and the $C_2$ is a discharged capacity after storage at 70°C for 60 hours.

9. The current collector according to claim 1, wherein an absolute value of a ΔR4 value according to Equation 4 below is 50% or more:

[Equation 4]

$$\Delta R4 = 100 \times (C_1\text{-}C_3)/C_1$$

wherein, the $C_1$ is a discharged capacity at 25°C, and the $C_3$ is a discharged capacity after storage at 130°C for 10 minutes.

10. The current collector according to claim 1, wherein the conductive copolymer has an oxidation potential in a range of 2.0 V to 5.0 V.

11. The current collector according to claim 1, wherein the conductive copolymer comprises a monomer unit of Formula 1 below:

[Formula 1]

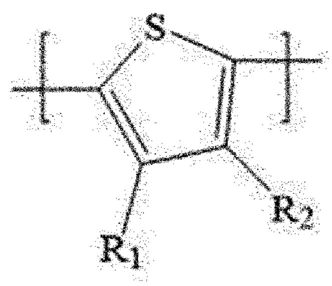

wherein, $R_1$ and $R_2$ are each independently hydrogen, a polar functional group, or a long-chain hydrocarbon functional group, with the proviso that at least one of $R_1$ and $R_2$ is a polar functional group or a long-chain hydrocarbon functional group; or
$R_1$ and $R_2$ are connected to each other to form a divalent functional group of Formula 2 below:

[Formula 2]

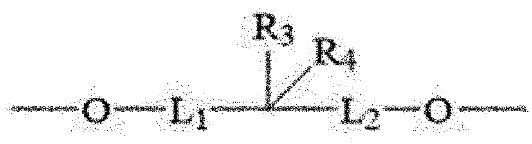

wherein, $L_1$ and $L_2$ are each independently a single bond, an alkylene group, or an alkylidene group, and $R_3$ and $R_4$ are each independently hydrogen, a polar functional group, or a long-chain hydrocarbon functional group, with the proviso that at least one of $R_3$ and $R_4$ is a polar functional group or a long-chain hydrocarbon functional group.

12. The current collector according to claim 11, wherein the polar functional group is a carboxyl group, a hydroxy group, an amino group, a cyano group, a nitro group, an ether group, or a functional group of Formula 3 below:

[Formula 3]

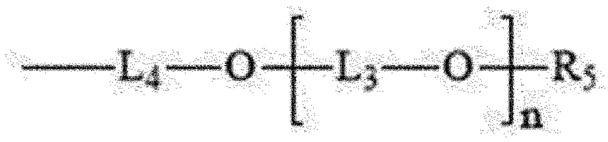

wherein, $L_4$ is a single bond, an alkylene group or an alkylidene group, $L_3$ is an alkylene group or an alkylidene group, $R_5$ is hydrogen or an alkyl group, and n is a number in a range of 1 to 10.

**13.** The current collector according to claim 1, wherein the conductive copolymer comprises a long-chain hydrocarbon functional group and a polar functional group.

**14.** The current collector according to claim 13, wherein a ratio M1/M2 of a mole number M1 of the long-chain hydrocarbon functional group relative to a mole number M2 of the polar functional group in the conductive copolymer is in a range of 1.5 to 20.

**15.** The current collector according to claim 1, wherein the conductive copolymer comprises a monomer unit of Formula 4 and a monomer unit of Formula 5:

[Formula 4]

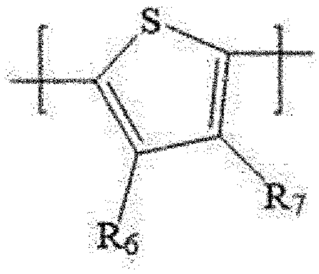

wherein, $R_6$ and $R_7$ are each independently hydrogen or a long-chain hydrocarbon functional group, with the proviso that at least one of $R_6$ and $R_7$ is a long-chain hydrocarbon functional group, or
$R_6$ and $R_7$ are connected to each other to form a divalent functional group of Formula 6 below:

[Formula 5]

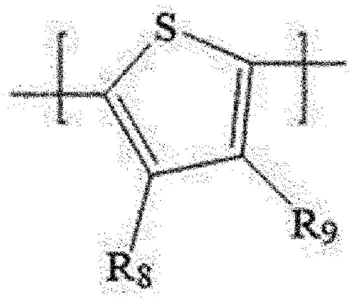

wherein, $R_8$ and $R_9$ are each independently hydrogen or a polar functional group, with the proviso that at least one of $R_8$ and $R_9$ is a polar functional group, or
$R_8$ and $R_9$ are connected to each other to form a divalent functional group of Formula 7 below:

[Formula 6]

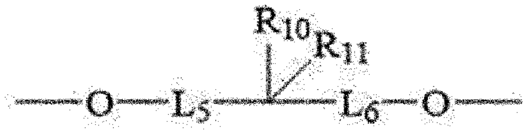

wherein, $L_5$ and $L_6$ are each independently a single bond, an alkylene group, or an alkylidene group, and $R_{10}$ and $R_{11}$ are each independently hydrogen or a long-chain hydrocarbon functional group, with the proviso that at least one of $R_{10}$ and $R_{11}$ is a long-chain hydrocarbon functional group:

[Formula 7]

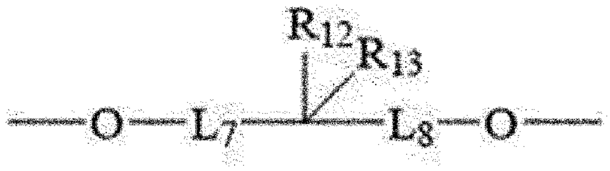

wherein, $L_7$ and $L_8$ are each independently a single bond, an alkylene group, or an alkylidene group, and $R_{12}$ and $R_{13}$ are each independently hydrogen or a polar functional group, with the proviso that at least one of $R_{12}$ and $R_{13}$ is a polar functional group.

**16.** An electrode, comprising:

the current collector of any one of claims 1 to 15; and
an active material layer formed on the polymer layer of the current collector.

**17.** An electrode assembly, comprising:
the electrode of claim 16.

**18.** A secondary battery, comprising:
the electrode current collector of claim 17.

Fig. 1

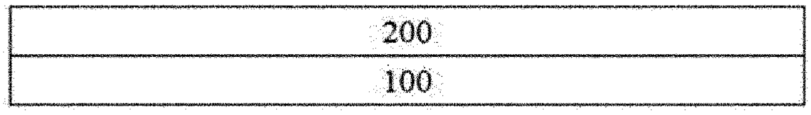


Fig. 2

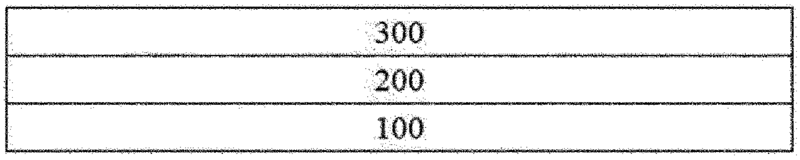


Fig. 3

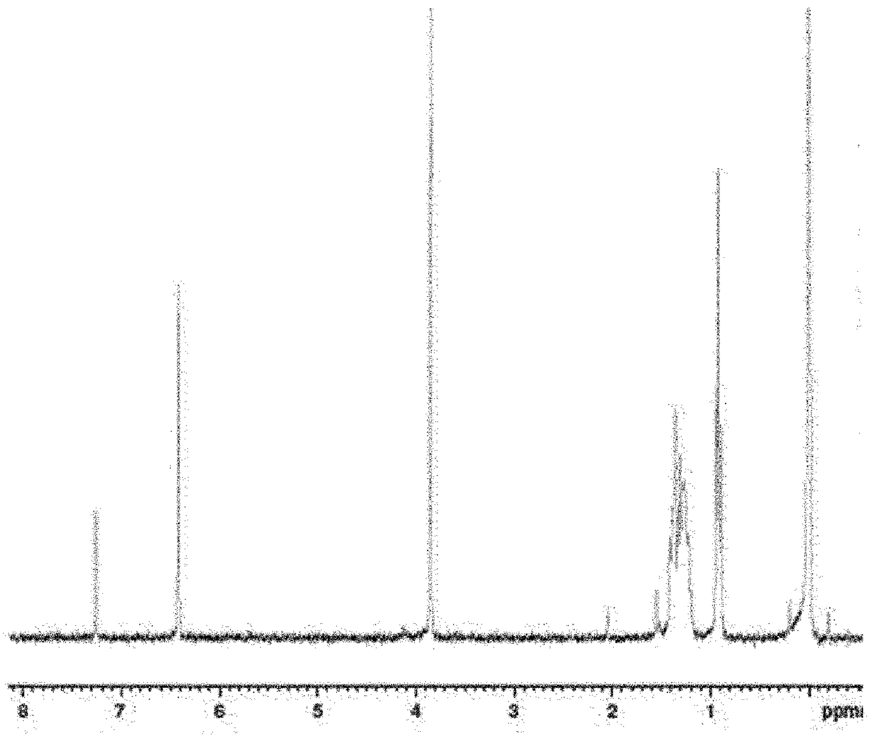

Fig. 4

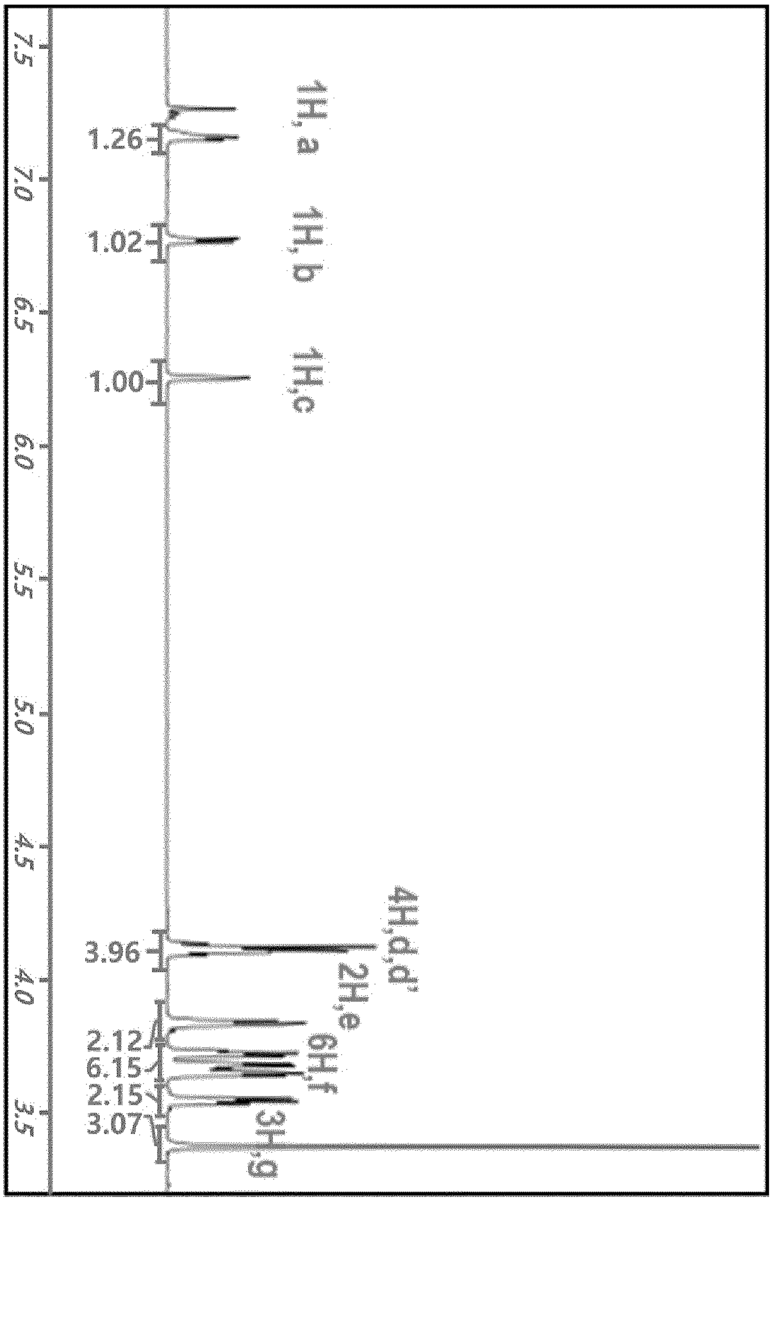

7.5
7.0
6.5
6.0
5.5
5.0
4.5
4.0
3.5
1.26
1.02
1.00
3.96
2.12
6.15
2.15
3.07
1H,a
1H,b
1H,c
4H,d,d'
2H,e
6H,f
3H,g

**INTERNATIONAL SEARCH REPORT**

International application No. **PCT/KR2023/011298**

**A. CLASSIFICATION OF SUBJECT MATTER**

**H01M 4/13**(2010.01)i; **H01M 4/66**(2006.01)i; **H01M 10/0525**(2010.01)i; **C08G 61/12**(2006.01)i; **C08L 65/00**(2006.01)i; **H01M 4/02**(2006.01)i

According to International Patent Classification (IPC) or to both national classification and IPC

**B. FIELDS SEARCHED**

Minimum documentation searched (classification system followed by classification symbols)

H01M 4/13(2010.01); C07D 333/10(2006.01); H01M 10/052(2010.01); H01M 10/42(2006.01); H01M 4/131(2010.01); H01M 4/134(2010.01); H01M 4/48(2010.01); H01M 4/64(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, CAplus) & keywords: 집전체(current collector), 전도성 고분자(conductive polymer), 폴리티오펜(polythiophene), 전극(electrode), 이차전지(secondary battery)

**C. DOCUMENTS CONSIDERED TO BE RELEVANT**

| Category* | Citation of document, with indication, where appropriate, of the relevant passages | Relevant to claim No. |
|---|---|---|
| X<br>Y | ZHANG, H. et al. Poly(3-butylthiophene)-based positive-temperature-coefficient electrodes for safer lithium-ion batteries. Electrochimica Acta. 2016, vol. 187, pp. 173-178.<br>See abstract; 2. Experimental section and 3. Results and discussion section; and figure 7(d). | 1-11,16-18<br>12-15 |
| Y | KR 10-2022-0043773 A (LG ENERGY SOLUTION, LTD.) 05 April 2022 (2022-04-05)<br>See claims 1 and 18; paragraphs [0033]-[0037] and [0048]; manufacturing examples 1, 2 and 4; and example 2. | 12-15 |
| X | KR 10-2001-0017407 A (SAMSUNG SDI CO., LTD.) 05 March 2001 (2001-03-05)<br>See claims 1, 3, 5 and 7; page 3, lines 20-41; and examples 1-4. | 1,16-18 |
| A | KR 10-2013-0082071 A (KABUSHIKI KAISHA HITACHI SEISAKUSHO(D/B/A HITACHI, LTD.)) 18 July 2013 (2013-07-18)<br>See entire document. | 1-18 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 November 2023** | **07 November 2023** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

INTERNATIONAL SEARCH REPORT

International application No.
PCT/KR2023/011298

C. DOCUMENTS CONSIDERED TO BE RELEVANT

| Category* | Citation of document, with indication, where appropriate, of the relevant passages | Relevant to claim No. |
|---|---|---|
| A | KR 10-2009-0127732 A (INDUSTRY-ACADEMIC COOPERATION FOUNDATION, YONSEI UNIVERSITY) 14 December 2009 (2009-12-14) See entire document. | 1-18 |
| PX | KR 10-2023-0037451 A (LG ENERGY SOLUTION, LTD.) 16 March 2023 (2023-03-16) See claims 1 and 7-10; paragraphs [0159]-[0162]; and example 1. | 1,11,16-18 |

Form PCT/ISA/210 (second sheet) (July 2022)

**INTERNATIONAL SEARCH REPORT**
**Information on patent family members**

International application No.
**PCT/KR2023/011298**

| Patent document cited in search report | | | Publication date (day/month/year) | Patent family member(s) | | | Publication date (day/month/year) |
|---|---|---|---|---|---|---|---|
| KR | 10-2022-0043773 | A | 05 April 2022 | CN | 116420247 | A | 11 July 2023 |
| | | | | EP | 4203094 | A1 | 28 June 2023 |
| | | | | WO | 2022-071704 | A1 | 07 April 2022 |
| KR | 10-2001-0017407 | A | 05 March 2001 | KR | 10-0573098 | B1 | 24 April 2006 |
| KR | 10-2013-0082071 | A | 18 July 2013 | CN | 103190020 | A | 03 July 2013 |
| | | | | JP | 5144832 | B1 | 13 February 2013 |
| | | | | KR | 10-1343477 | B1 | 19 December 2013 |
| | | | | US | 2013-0252095 | A1 | 26 September 2013 |
| | | | | US | 8877385 | B2 | 04 November 2014 |
| | | | | WO | 2013-065141 | A1 | 10 May 2013 |
| KR | 10-2009-0127732 | A | 14 December 2009 | KR | 10-1007864 | B1 | 14 January 2011 |
| KR | 10-2023-0037451 | A | 16 March 2023 | WO | 2023-038474 | A1 | 16 March 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020220096262